(19) **Europäisches Patentamt / European Patent Office / Office européen des brevets**

(11) **EP 3 189 835 B1**

(12) **EUROPEAN PATENT SPECIFICATION**

(45) Date of publication and mention
of the grant of the patent:
**25.07.2018 Bulletin 2018/30**

(51) Int Cl.:
***A61K 31/167*** *(2006.01)*      ***A61K 38/22*** *(2006.01)*
***A61P 25/00*** *(2006.01)*

(21) Application number: **17153823.4**

(22) Date of filing: **27.02.2012**

(54) **MEDICAL AGENT FOR SUPPRESSING MALIGNANT TUMOR METASTASIS**

MEDIZINISCHER WIRKSTOFF ZUR HEMMUNG MALIGNER TUMORMETASTASEN

AGENT MÉDICAL DE SUPPRESSION DE MÉTASTASE DE TUMEURS MALIGNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2011 JP 2011041263**

(43) Date of publication of application:
**12.07.2017 Bulletin 2017/28**

(62) Document number(s) of the earlier application(s) in
accordance with Art. 76 EPC:
**12752973.3 / 2 682 128**

(73) Proprietors:
• **National Cerebral and Cardiovascular Center Suita-shi**
**Osaka 565-8565 (JP)**
• **OSAKA UNIVERSITY**
**Suita-shi, Osaka 565-0871 (JP)**
• **Shionogi & Co., Ltd.**
**Osaka 541-0045 (JP)**

(72) Inventors:
• **KANGAWA, Kenji**
**Suita-shi, Osaka 565-8565 (JP)**
• **HOSODA, Hiroshi**
**Suita-shi, Osaka 565-8565 (JP)**
• **NOJIRI, Takashi**
**Suita-shi, Osaka 565-0871 (JP)**
• **OKUMURA, Meinoshin**
**Suita-shi, Osaka 565-0871 (JP)**

(74) Representative: **Vossius & Partner**
**Patentanwälte Rechtsanwälte mbB**
**Siebertstrasse 3**
**81675 München (DE)**

(56) References cited:
**EP-A1- 0 497 368**      **WO-A1-02/078683**
**WO-A2-01/80890**      **US-A1- 2005 209 139**
**US-A1- 2005 272 650**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> National cerebral and cardiovascular center Osaka University Shionogi & Co., Ltd.

<120> Medicinal agent for suppressing malignant tumor metastasis

<130> W2546 EP/1

<140> EP 17 15 3823.4
<141> 2012-02-27

<150> JP 2011-41263
<151> 2011-2-28

<160> 11

<170> PatentIn version 3.4

<210> 1
<211> 28
<212> PRT
<213> Homo sapiens

<400> 1

```
    Ser Leu Arg Arg Ser Ser Cys Phe Gly Gly Arg Met Asp Arg Ile Gly
    1               5                   10                  15


    Ala Gln Ser Gly Leu Gly Cys Asn Ser Phe Arg Tyr
                20                  25
```

<210> 2
<211> 28
<212> PRT
<213> Rattus rattus

<400> 2

```
    Ser Leu Arg Arg Ser Ser Cys Phe Gly Gly Arg Ile Asp Arg Ile Gly
    1               5                   10                  15


    Ala Gln Ser Gly Leu Gly Cys Asn Ser Phe Arg Tyr
                20                  25
```

<210> 3
<211> 32
<212> PRT
<213> Homo sapiens

<400> 3

```
        Ser Pro Lys Met Val Gln Gly Ser Gly Cys Phe Gly Arg Lys Met Asp
        1               5                   10                  15


        Arg Ile Ser Ser Ser Ser Gly Leu Gly Cys Lys Val Leu Arg Arg His


            20                  25                  30
```

<210> 4
<211> 32
<212> PRT
<213> Sus scrofa

<400> 4

```
        Ser Pro Lys Thr Met Arg Asp Ser Gly Cys Phe Gly Arg Arg Leu Asp
        1               5                   10                  15


        Arg Ile Gly Ser Leu Ser Gly Leu Gly Cys Asn Val Leu Arg Arg Tyr
                20                  25                  30
```

<210> 5
<211> 45
<212> PRT
<213> Rattus rattus

<400> 5

```
        Ser Gln Asp Ser Ala Phe Arg Ile Gln Glu Arg Leu Arg Asn Ser Lys
        1               5                   10                  15


        Met Ala His Ser Ser Ser Cys Phe Gly Gln Lys Ile Asp Arg Ile Gly
                20                  25                  30


        Ala Val Ser Arg Leu Gly Cys Asp Gly Leu Arg Leu Phe
                35                  40                  45
```

<210> 6
<211> 21
<212> PRT
<213> Artificial

<220>
<223> Ring structure sequence of ANPs and BNPs

<220>
<221> misc-feature
<222> (4)..(5)
<223> Xaa can be any naturally occurring amino acid or artificial amino
acid mimetic

<220>
<221> misc-feature
<222> (6)

<223> Xaa can be Met, Leu or Ile

<220>
<221> misc-feature
<222> (10)..(14)
<223> Xaa can be any naturally occurring amino acid or artificial amino
acid mimetic

<220>
<221> misc-feature
<222> (18)..(20)
<223> Xaa can be any naturally occurring amino acid or artificial amino
acid mimetic

<400> 6

```
Cys Phe Gly Xaa Xaa Xaa Asp Arg Ile Xaa Xaa Xaa Xaa Xaa Leu Gly
1               5               10              15

Cys Xaa Xaa Xaa Arg
                20
```

<210> 7
<211> 22
<212> PRT
<213> Homo sapiens

<400> 7

```
Gly Leu Ser Lys Gly Cys Phe Gly Leu Lys Leu Asp Arg Ile Gly Ser
1               5               10              15

Met Ser Gly Leu Gly Cys
                20
```

<210> 8
<211> 53
<212> PRT
<213> Homo sapiens

<400> 8

```
Asp Leu Arg Val Asp Thr Lys Ser Arg Ala Ala Trp Ala Arg Leu Leu
1               5               10              15

Gln Glu His Pro Asn Ala Arg Lys Tyr Lys Gly Ala Asn Lys Lys Gly
                20              25              30

Leu Ser Lys Gly Cys Phe Gly Leu Lys Leu Asp Arg Ile Gly Ser Met
                35              40              45

Ser Gly Leu Gly Cys
    50
```

<210> 9
<211> 22
<212> PRT
<213> Gallus gallus

<400> 9

```
Gly Leu Ser Arg Ser Cys Phe Gly Val Lys Leu Asp Arg Ile Gly Ser
1               5                   10                  15

            Met Ser Gly Leu Gly Cys
                        20
```

<210> 10
<211> 22
<212> PRT
<213> Rana sp.

<400> 10

```
Gly Tyr Ser Arg Gly Cys Phe Gly Val Lys Leu Asp Arg Ile Gly Ala
1               5                   10                  15

Phe Ser Gly Leu Gly Cys
            20
```

<210> 11
<211> 17
<212> PRT
<213> Artificial

<220>
<223> Ring structure sequence of CNP

<220>
<221> misc-feature
<222> (11)..(11)
<223> Xaa can be any naturally occurring amino acid or artificial amino acid mimetics, preferably Ser or Ala

<220>
<221> misc_feature
<222> (12)..(12)
<223> Xaa can be any naturally occurring amino acid or artificial amino acid mimetics, preferably Met, Phe or Glu

<400> 11

```
Cys Phe Gly Leu Lys Leu Asp Arg Ile Gly Xaa Xaa Ser Gly Leu Gly
1               5                   10                  15

Cys
```

**Description**

Technical Field

The present invention relates to a medicinal composition for suppressing the metastasis of a malignant tumor including carcinoma, the composition comprising, as an active ingredient, a vascular endothelial intracellular cGMP enhancer represented by natriuretic peptide receptor GC-B agonists. The invention is defined in the claims.

Background Art

Malignant tumors represented by carcinoma are diseases caused by the abnormal growth of cells, and the most distinctive characteristic of malignant tumors is invasion into the surrounding tissue and metastasis to other organs. It has been long known that the leading cause of death for malignant tumor patients is not the growth of the primary tumor but multiple organ failure resulting from distant metastasis of the tumor cells. Thus, control of malignant tumor metastasis is one of the most crucial issues in the whole area of cancer treatment but has not yet been achieved so far.

Metastasis of an epithelial malignant tumor (carcinoma) is considered to be caused by various physiological phenomena of cancer cells, such as the acquisition of motility and migrating ability through epithelial to mesenchymal transition (hereinafter, abbreviated to "EMT"), invasion into the surrounding tissue, migration and invasion into blood vessels and lymphatic vessels, colonization in distant tissue, metastatic lesion formation, etc. In recent years, EMT has particularly attracted attention. In normal epithelial tissue, cells are tightly connected together via adhesion molecules to form tissue, but in the EMT phenomenon, cells lose intercellular adhesion ability with the progression of malignancy of cancer and are transformed to acquire migration ability and motility. In the process of EMT, the expression of E cadherin decreases and the expression of N cadherin increases. Therefore, the gene expression ratio of N cadherin/E cadherin can serve as an indicator of EMT. Cancer cells that have acquired migration ability and motility in EMT become capable of migrating from the primary tumor into the surrounding tissue, blood vessels, and lymphatic vessels, and for this reason can be a trigger for cancer metastasis (Non Patent Literature 1: Thomas R. Geiger et al., Biochim. Biophys. Acta., 2009, 293-308). Also in a non-epithelial malignant tumor (sarcoma etc.), tumor cells that have become malignant and acquired motility and migrating ability invade into blood vessels etc., colonize the vascular endothelium of remote tissue to invade the tissue and then form a metastatic lesion. Thus, the metastasis of a malignant tumor proceeds by a mechanism completely different from that of tumor cell growth, and therefore cannot be sufficiently suppressed with the use of existing medicinal agents having a cell killing effect or a growth-suppressing activity. In addition, these medicinal agents generally have harmful effects on normal tissue, and chronic dosing thereof causes problems of serious side effects. For control of malignant tumor metastasis, prolonged administration is usually needed. Therefore there is a need to find and develop a safe medicinal agent for suppressing metastasis.

Intracellular cGMP is widely known as a second messenger, which is responsible for mediating in vivo signaling, and particularly well examined regarding the control of vascular smooth muscles. It is generally known that increase in intracellular cGMP in vascular smooth muscle cells relaxes smooth muscles and decreases blood pressure. Representative examples of medicinal agents having such effects include natriuretic peptides.

Natriuretic peptides include atrial natriuretic peptide (ANP), brain natriuretic peptide (BNP), and C-type natriuretic peptide (CNP). These peptides are known to bind specifically to transmembrane receptors having an intracellular guanylate cyclase domain and increase intracellular cGMP to express various physiological activities. The receptors have two types, namely natriuretic peptide receptor GC-A (also known as NPR-A) and natriuretic peptide receptor GC-B (also known as NPR-B), and it is known that ANP and BNP specifically bind to GC-A, and CNP specifically binds to GC-B (Non Patent Literature 2: Silver MA, Curr. Opin. Nephrol. Hypertens., 2006, vol. 15, 14-21).

ANP is a 28-amino acid peptide having a cyclic structure and is produced in and secreted from atrial cells. The peptide shows diuretic action in the kidney, and relaxes and dilates vascular smooth muscles in blood vessels. In addition, ANP antagonizes the actions of the renin-angiotensin-aldosterone system and vasopressin. These actions comprehensively reduce the load on the heart through lowering the blood pressure, body fluid volume, etc. BNP is a 32-amino acid peptide having a cyclic structure. It was first found in the brain, but later research revealed that the peptide is produced and secreted mainly in cardiomyocytes rather than the brain. The peptide has similar actions to those of ANP. ANP and BNP specifically bind to GC-A and thereby promote production of cGMP to express the above-mentioned actions (Non Patent Literature 3: Yoshibayashi M. et al., Eur. J. Endocrinol., 1996, vol. 135, 265-268). Indeed, the secretion of ANP is promoted with elevation of atrial filling pressure in congestive cardiac failure etc., and ANP alleviates the symptoms of congestive cardiac failure etc. via the above-mentioned actions. Human ANP (hANP) is clinically used as a therapeutic agent for acute cardiac failure in Japan. Also, the secretion of BNP is increased in cardiac failure patients, and BNP alleviates various symptoms associated with cardiac failure via the above-mentioned actions. Human BNP (hBNP) is approved as a therapeutic agent for acute cardiac failure in the United States etc.

It is known that ANP and BNP have various physiological activities besides blood-pressure regulating actions including diuretic action, vasodilating action, etc. For example, the actions of ANP on bacterial infection-induced inflammation and associated failure in the endothelial barrier function have been reported (for example, Non Patent Literature 4: Xung J., et al., J. appl. Physiol., 2011, vol. 110 (1), 213-224, etc.) . Regarding malignant tumors, experimental reports have been submitted by Vesely et al. on the growth-suppressing effect of ANP on cancer cells (for example, Non Patent Literature 5: Vesely BA et al., Eur J Clin Invest., 2005, vol. 35, 60-69 etc.) . These reports indicate that, in addition to ANP, long acting natriuretic peptide, vessel dilator, kalliuretic peptide, etc., of which the amino acid sequences are not likely to contribute to binding to the natriuretic peptide receptor GC-A, have the same or stronger growth-suppressing activity on cancer cells as compared to the growth-suppressing activity of ANP whereas BNP does not have such a growth-suppressing activity. From this, it is considered that the growth-suppressing activity of ANP on cancer cells reported by Vesely et al. is not based on the agonist activity for GC-A. The actions of ANP and BNP related to the metastasis of tumor cells are still unknown even after the release of the reports by Vesely et al. and others.

CNP is a physiologically active peptide found in porcine brain, and it is known that mammals have in their bodies a 22-amino acid peptide CNP-22 and a 53-amino acid peptide CNP-53, which is an N-terminally elongated form of CNP-22. CNP was originally considered to function as a neuropeptide, but the following research revealed that the peptide also exists in peripheral tissues and plays an important role in the process of bone growth. It has been confirmed so far that CNP controls the differentiation and growth of chondrocytes mainly in the growth plate cartilage, and therefore CNP is a promising therapeutic agent for dwarfism including achondroplasia. CNP is known to have various physiological activities besides those in bone and cartilage. Examples thereof include actions on blood vessels, such as growth suppression of vascular smooth muscle cells, growth promotion of vascular endothelial cells, etc. However, the action of CNP on the metastasis of malignant tumors is unknown.

Known examples of other medicinal agents that increase intracellular cGMP concentration include inhibitors of phosphodiesterase (PDE) 5, which is a cGMP-degrading enzyme, such as sildenafil citrate; NO donors, such as nitroglycerin; eNOS substrates, such as arginine; inhibitors of neutral endopeptidase (NEP), which degrades ANP and BNP; etc. In addition, guanylin, uroguanylin, etc., which are agonists of guanylate cyclase C (GC-C) known to have an action in the digestive tract, also increase intracellular cGMP concentration. These medicinal agents are also known to relate to various physiological phenomena including inflammatory reactions and allergic reactions, and in some recent reports describe that the medicinal agents also relates to the control of tumor cells. However, it is unknown that these agents act on vascular endothelial cells to suppress the metastasis of tumor cells under actual physiological conditions. For example, regarding sildenafil, while sildenafil is reported to have a suppressing effect on malignant tumors (for example, Non Patent Literature 6: Das et al., Proc. Natl. Acad. Sci., 2010, vol. 107 (42), 18202-18207 etc.), it is also reported to have no effect on the growth or metastasis of tumor cells (for example, Non Patent Literature 7: Dian C. N., et al., Journal of Urology, 2003, vol. 170 (3), 994-997) . Regarding GC-C, it is known to be overexpressed in colorectal cancer cells and to relate to MMP9 production by the cancer cells (for example, Non Patent Literature 8: Lubbe W. J., et al., Cancer Res., 2009, vol. 69 (8), 3529-3536). Regarding NO, there is a report that NO inhibits tumor cell adhesion to isolated postcapillary venules of LPS-treated rats (unilobar) (Non Patent Literature 9: Kong L., et al., Clin Exp. Metastasis, 1996, vol. 14 (3), 335-343) . However, this experiment was conducted with the use of LPS, which is a strong inflammation inducer, and thus is considered to be performed under the conditions that are quite different from physiological phenomena in actual tumor metastasis. In the following dozen years or so, no follow-up report was released, and the physiological action of NO on tumor metastasis remains unknown.

However, it is unknown that these medicinal agents act on vascular endothelial cells to suppress the metastasis of tumor cells.

Citation List

Non Patent Literature

[NPL 1] Thomas R. Geiger et al., Biochim. Biophys. Acta., 2009, 293-308
[NPL 2] Silver MA, Curr. Opin. Nephrol. Hypertens., 2006, vol. 15, 14-21
[NPL 3] Yoshibayashi M. et al., Eur. J. Endocrinol., 1996, vol. 135, 265-268
[NPL 4] Xung J., et al., J. appl. Physiol., 2011, vol. 110 (1), 213-224
[NPL 5] Vesely BA et al., Eur J Clin Invest., 2005, vol. 35, 60-69
[NPL 6] Das et al., Proc. Natl. Acad. Sci., 2010, vol. 107 (42), 18202-18207
[NPL 7] Dian C. N., et al., Journal of Urology, 2003, vol. 170 (3), 994-997
[NPL 8] Lubbe W. J., et al., Cancer Res., 2009, vol. 69 (8), 3529-3536
[NPL 9] Kong L., et al., Clin Exp. Metastasis, 1996, vol. 14 (3), 335-343

Summary of Invention

Technical Problem

An objective of the present invention is to provide a safe medicinal agent for suppressing the metastasis of a malignant tumor including carcinoma.

Solution to Problem

The present inventors made extensive research on medicinal agents for suppressing the metastasis of cancer and found that the natriuretic peptide receptor GC-A agonist exerts the action of suppressing EMT of cancer cells and thereby suppressing the acquisition by cancer cells of the activities associated with metastasis, such as migration ability, motility, and invasive ability; induces cancer cell-specific apoptosis; and also acts on vascular endothelial cells in the hosts, and thereby the agonist suppresses the implantation/metastasis of tumor cells. Further, the inventors found in the clinical study that in cancer patients who received 3-day administration of hANP at sufficiently low dosage not to affect blood pressure after surgical cancer resection, recurrence of the cancer was significantly suppressed. In addition, in a metastasis test where tumor cells were injected into the tail vein of mice, it was confirmed that administration of a GC-A agonist significantly inhibited tumor metastasis in not only GC-A expressing tumor cells but also GC-A non-expressing tumor cells, such as B6 melanoma. Further, in the same metastasis test conducted using endothelial tissue-specific GC-A gene knockout mice, the metastasis of tumors was significantly increased as compared to that of wild type mice. Conversely, in the same metastasis test conducted using mice in which the GC-A gene was overexpressed in endothelial cells, the metastasis of tumors was significantly suppressed as compared to that of wild type mice. These results suggest that the signals mediated by GC-A expressed in endothelial tissue play a central role in the metastasis of tumor cells. Further, in the same metastasis test conducted with the administration of medicinal agents including a GC-B agonist such as CNP, a PDE5 inhibitor such as sildenafil citrate, etc., which are medicinal agents that increase intracellular cGMP by mechanisms other than the mechanism through GC-A, significant metastasis-suppressing effects were observed. Based on these findings, the present inventors conducted further research and completed the present invention.

That is, natriuretic peptide receptor GC-A agonists directly act on tumor cells expressing GC-A to suppress EMT and resulting migration ability and invasion ability. In addition, an indirect metastasis-suppressing effect is exhibited by agonists that increase the intracellular cGMP level to induce signaling (for example, a GC-A agonist, a GC-B agonist, a PDE5 inhibitor, etc.) through their actions on the endothelial cells of a blood vessel or a lymphatic vessel to prevent tumor cells from adhering to and infiltrating into the vascular endothelium, regardless of whether the tumor cells are expressing GC-A or not. Since GC-A and GC-B are usually expressed in endothelial cells, this indirect effect can suppress the metastasis of tumors regardless of the type of tumor.

The present invention includes the following aspects.

The present invention relates to a medicinal agent for suppressing or preventing the metastasis of a malignant tumor including carcinoma, the agent comprising, as an active ingredient, at least one kind of agent which increases intracellular cGMP concentration in vascular endothelium (referred to as "vascular endothelial intracellular cGMP enhancer") (for example, the natriuretic peptide receptor GC-A agonist).

Also, the present disclosure relates to a method for preventing or suppressing the metastasis of a malignant tumor including carcinoma, the method comprising administering an effective amount of at least one kind of vascular endothelial intracellular cGMP enhancer (for example, the natriuretic peptide receptor GC-A agonist) to a patient in need of controlling the metastasis of a malignant tumor including carcinoma.

Also, the present invention relates to at least one kind of vascular endothelial intracellular cGMP enhancer (for example, the natriuretic peptide receptor GC-A agonist) for use in preventing or suppressing the metastasis of a malignant tumor including carcinoma.

Regarding the medicinal agent, therapeutic method, etc. provided by the present invention (hereinafter referred to as "medicinal agent etc."), the vascular endothelial intracellular cGMP enhancer is, preferably, a natriuretic peptide receptor GC-A agonist, a natriuretic peptide receptor GC-B agonist, a GC-C agonist, a NEP inhibitor, a PDE5 inhibitor, a NO donor, an eNOS activator, a cGMP analog, etc. and is a substance having an activity of acting on vascular endothelial cells to increase the intracellular cGMP concentration. More preferred are a natriuretic peptide receptor GC-A agonist, a natriuretic peptide receptor GC-B agonist, a NEP inhibitor, and a PDE5 inhibitor, and further more preferred is a natriuretic peptide receptor GC-A agonist. The vascular endothelial intracellular cGMP enhancer of the present invention, which at least has an activity of acting on the endothelial cells of a blood vessel or a lymphatic vessel to increase the intracellular cGMP concentration, preferably increases peripheral vascular intracellular cGMP concentration when administered to a living body.

In the medicinal agent etc. provided by the present invention, the natriuretic peptide receptor GC-A agonist is preferably any one selected from the following (a1) to (a6) or pharmacologically acceptable salt thereof and is a substance having an agonist activity for the natriuretic peptide receptor GC-A: (a1) atrial natriuretic peptide; (a2) brain natriuretic peptide; a substance comprising an active fragment of (a1) or (a2); (a4) a mutant having substitution, deletion, insertion, and/or addition of one to several amino acids in any one of the amino acid sequences of (a1) to (a3); (a5) a derivative of any one of (a1) to (a4) ; and (a6) a modified form of any one of (a1) to (a5), or a pharmacologically acceptable salt thereof.

Here, further preferred examples of the natriuretic peptide receptor GC-A agonist are shown below.

The atrial natriuretic peptide (a1) is preferably a peptide consisting of the amino acid sequence of SEQ ID NO: 1 or 2 in the sequence listing.

The brain natriuretic peptide (a2) is preferably a peptide consisting of the amino acid sequence of SEQ ID NO: 3, 4, or 5 in the sequence listing.

The active fragment (a3) is preferably a peptide having the amino acid sequence of SEQ ID NO: 6 in the sequence listing, and more preferably a peptide consisting of the amino acid sequence from position 7 to position 27 of SEQ ID NO: 1 or 2 in the sequence listing, the amino acid sequence from position 10 to position 30 of SEQ ID NO: 3 or 4, or the amino acid sequence from position 23 to position 43 of SEQ ID NO: 5 in the sequence listing.

The mutant (a4) preferably consists of any of the amino acid sequences of SEQ ID NOs: 1 to 5 in the sequence listing having substitution, deletion, insertion, and/or addition of one to several amino acids at one to several positions other than the amino acids shown in SEQ ID NO: 6 in the sequence listing. More preferably, the mutant is (i) a peptide consisting of the amino acid sequence of SEQ ID NO: 1 or 2 having substitution, deletion, insertion, and/or addition of one to several amino acids at any one to several positions selected from positions 1 to 6 and 28, (ii) a peptide consisting of the amino acid sequence of SEQ ID NO: 3 or 4 having substitution, deletion, insertion, and/or addition of one to several amino acids at any one to several positions selected from positions 1 to 9, 31 and 32, or (iii) a peptide consisting of the amino acid sequence of SEQ ID NO: 5 having substitution, deletion, insertion, and/or addition of one to several amino acids at any one to several positions selected from positions 1 to 22, 44 and 45.

The derivative (a5) preferably comprises any one of the amino acid sequences of SEQ ID NOs: 1 to 5 of the sequence listing, or the above-mentioned active fragment (a3), and more preferably further comprises at least one of the Fc region of an immunoglobulin, a serum albumin, and the C terminus of ghrelin.

The modified form (a6) preferably comprises any one of the amino acid sequences of SEQ ID NOs: 1 to 5 of the sequence listing, and at least one amino acid other than the amino acids in SEQ ID NO: 6 is chemically modified. More preferably, the modified form is prepared by chemical modification by binding of a polymer, such as PEG, PVA, etc. used in the pharmaceutical field.

In the medicinal agent etc. provided by the present invention, the natriuretic peptide receptor GC-A agonist may be an anti-GC-A antibody. Such an anti-GC-A antibody may be one which specifically binds to GC-A and has an agonist activity for GC-A. The antibody may be a polyclonal antibody or a monoclonal antibody, but preferably a humanized monoclonal anti-GC-A antibody or a human monoclonal anti-GC-A antibody.

In the medicinal agent etc. provided by the present invention, the natriuretic peptide receptor GC-B agonist is preferably any one selected from the following (b1) to (b5), and is a substance having an agonist activity for the natriuretic peptide receptor GC-B: (b1) a C-type natriuretic peptide; (b2) a substance comprising an active fragment of (b1) ; (b3) a mutant having substitution, deletion, insertion, and/or addition of one to several amino acids in the amino acid sequence of (b1) or (b2) ; (b4) a derivative of any one of (b1) to (b3); and (b5) a modified form of any one of (b1) to (b4), or a pharmacologically acceptable salt thereof, or a pharmacologically acceptable salt thereof.

Here, further preferred examples of the natriuretic peptide receptor GC-B agonist are shown below.

The C-type natriuretic peptide (b1) is preferably a peptide consisting of the amino acid sequence of SEQ ID NO: 7 (hCNP-22), SEQ ID NO: 8 (hCNP-53), SEQ ID NO: 9 (CNP of fowl origin), or SEQ ID NO: 10 (CNP of frog origin) in the sequence listing.

The active fragment (b2) is preferably a peptide having the amino acid sequence of SEQ ID NO: 11 in the sequence listing, and more preferably a peptide consisting of the amino acid sequence from position 6 to position 22 of the amino acid sequence of SEQ ID NO: 7 (hCNP6-22) in the sequence listing.

The mutant (b3) consists of any of the amino acid sequences of SEQ ID NOs : 7 to 10 having substitution, deletion, insertion, and/or addition of one to several amino acids at one to several positions other than the amino acids shown in SEQ ID NO: 11. More preferably, the mutant is (i) a peptide consisting of the amino acid sequence of SEQ ID NO: 7, 9, or 10 having substitution, deletion, insertion, and/or addition of one to several amino acids at any one to several positions selected from positions 1 to 5, or (ii) a peptide consisting of the amino acid sequence of SEQ ID NO: 8 having substitution, deletion, insertion, and/or addition of one to several amino acids at any one to several positions selected from positions 1 to 36.

The derivative (b4) preferably comprises any one of the amino acid sequences of SEQ ID NOs: 7 to 11 of the sequence listing, and/or further comprises at least one of a Fc region of an immunoglobulin, a serum albumin, and the C terminus of ghrelin.

The modified form (b5) preferably comprises any one of the amino acid sequences of SEQ ID NOs: 7 to 10 of the sequence listing, and at least one amino acid other than the amino acids shown in SEQ ID NO: 11 is chemically modified. More preferably, the modified form is prepared by chemical modification by addition of a polymer used in the pharmaceutical field.

In the medicinal agent etc. provided by the present invention, the natriuretic peptide receptor GC-B agonist may be an anti-GC-B antibody. Such an anti-GC-B antibody may be one which specifically binds to GC-B and has an agonist activity for GC-B. The antibody may be a polyclonal antibody or a monoclonal antibody, but preferably a humanized monoclonal anti-GC-B antibody or a human monoclonal anti-GC-B antibody.

As the PDE5 inhibitor used for the medicinal agent etc. of the present invention, various compounds, such as sildenafil, vardenafil, tadalafil, udenafil, mirodenafil, etc., or a pharmacologically acceptable salt thereof may be used, and preferred is sildenafil citrate, vardenafil hydrochloride, or tadalafil.

In the medicinal agent etc. provided by the present invention, the vascular endothelial intracellular cGMP enhancer, for example, a natriuretic peptide receptor GC-A agonist may be administered in such a dosage as not to largely change at least one of the blood pressure, the heart rate, and the urine volume of the patient. The dosage may be, for example in the cases of continuous administration of a natriuretic peptide receptor GC-A agonist or GC-B agonist, 0.1 μg/kg/min or less, preferably 0.05 μg/kg/min or less, and more preferably 0.025 μg/kg/min or less. The period of the continuous administration is usually one day or longer, and preferably one to several days.

The medicinal agent etc. provided by the present invention can be used in the form where a patient who undergoes surgery for removal of a malignant tumor receives administration for a period appropriate for the surgery (for example, for several days from the day before the surgery) . The administration method is, for example, as follows: a natriuretic peptide receptor GC-A agonist or a natriuretic peptide receptor GC-B agonist is continuously administered in a dosage of 0.1 μg/kg/min or less from before the surgery for a certain period of time after surgery; preferably a peptide consisting of any of the amino acid sequence of SEQ ID NOs: 1 to 5 and 7 to 10 is continuously administered in a dosage of 0.05 μg/kg/min or less from before the tumor-removing surgery for a certain period of time after surgery (preferably not longer than 5 days) ; and more preferably a peptide consisting of the amino acid sequence of SEQ ID NO: 1 is continuously administered in a dosage of 0.025 μg/kg/min from before the tumor resection surgery for 3 days after surgery.

Regarding the medicinal agent etc. provided by the present invention, an embodiment in which two or more kinds of vascular endothelial intracellular cGMP enhancers as active ingredients are administered in combination is also included in the present invention. (The two or more kinds may be blended into a single formulation or separately formulated and administered simultaneously or at different timings.) A preferred combination is, for example, a combination of a natriuretic peptide receptor GC-A agonist or a natriuretic peptide receptor GC-B agonist with a PDE5 inhibitor or a NO donor, and a more preferred combination is a peptide consisting of the an amino acid sequence from position 6 to position 22 of SED ID NO: 1, 3, or 7 with sildenafil.

The present invention also provides a medicinal agent, a therapeutic method, etc. associated with administration of a vascular endothelial intracellular cGMP enhancer (for example, a natriuretic peptide receptor GC-A agonist), which acts on vascular endothelial cells in the body of a patient and exerts a pharmacological effect of preventing tumor cells from adhering to and infiltrating into vascular endothelial tissue.

Further, the medicinal agent etc. provided by the present invention includes a medicament which comprises a natriuretic peptide receptor GC-A agonist as an intracellular cGMP enhancer in vascular endothelial cells and which is administered to a subject at risk of developing an epithelial malignant tumor (preferably GC-A is expressed in the tumor cells of the subject). In this case, it is expectable that the pharmacological effect on tumor cells expressing GC-A (effect of suppressing the acquisition of metastatic ability) works synergistically with the effect on the vascular endothelium. The pharmacological effect on tumor cells may be at least one selected from suppression of EMT of tumor cells, suppression of acquisition of migration ability by tumor cells, suppression of acquisition of motility by tumor cells, suppression of invasion by tumor cells, and induction of cancer cell-specific apoptosis; and preferably two or more thereof.

Advantageous Effects of Invention

The medicinal agent of the present invention for suppressing or preventing the metastasis of a malignant tumor, the agent comprising, as an active ingredient, a vascular endothelial intracellular cGMP enhancer such as a natriuretic peptide receptor GC-A agonist, has an excellent effect that the agent acts on the endothelial cells of a blood vessel or a lymphatic vessel to prevent tumor cells from adhering to and migrating into vascular endothelium and thereby suppresses or prevents the metastasis of tumors regardless of the type of tumor. In particular, a medicinal agent comprising a natriuretic peptide receptor GC-A agonist as a vascular endothelial intracellular cGMP enhancer has an exceptionally excellent effect that the agent acts on tumor cells expressing GC-A and suppresses metastasis-related processes, such as EMT, migration activity, and invasion activity, that is, the agent can suppress the metastasis of a malignant tumor at multiple stages. With these excellent effects, the present invention can not only prevent the metastasis of a malignant tumor and recurrence after therapeutic resection of a tumor but also effectively suppress or prevent the metastasis of a malignant tumor that is hard to resect. Since sildenafil, hANP, hBNP, etc. have already been administered to many patients in clinical practice and the safety has been confirmed, the medicinal agent provided by the present invention has less risk of side effects and has an excellent tumor metastasis suppressing effect.

Brief Description of Drawings

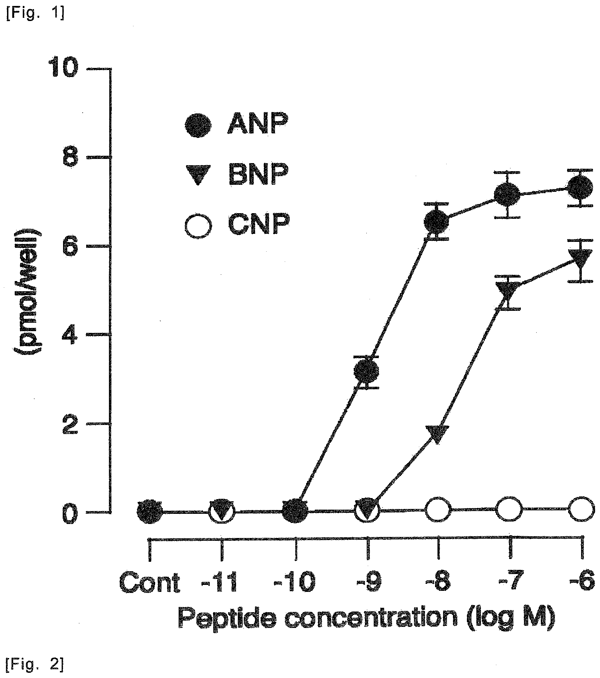

Fig. 1 shows a graph showing changes in the intracellular cGMP level responding to stimulation of A549 cells by natriuretic peptides (hANP (black circle), hBNP (down-pointing black triangle), and hCNP (white circle)) . The sample size at each point of the graph is n = 5 to 7.

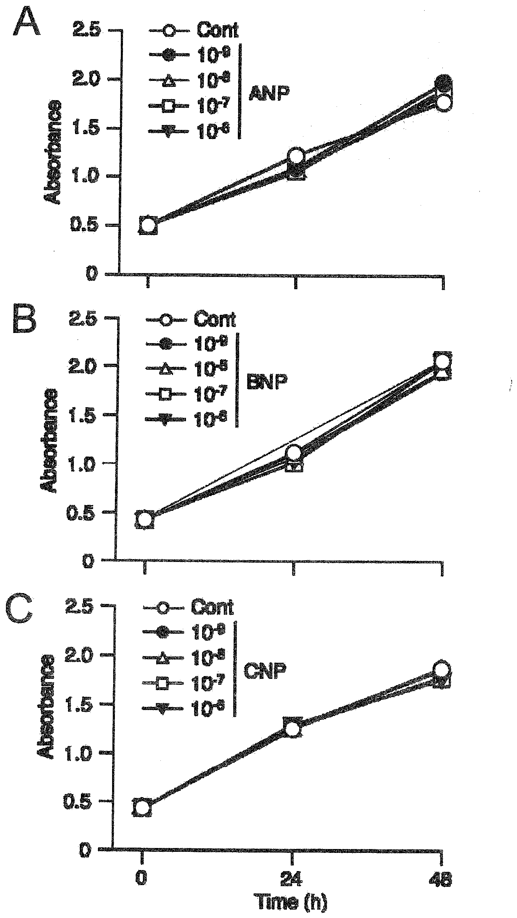

Fig. 2 shows graphs showing the growth of A549 cells in the presence or absence of hANP (Fig. 2A), hBNP (Fig. 2B), and hCNP (Fig. 2C) at each concentration based on the number of viable cells counted up to 48 hours of culture time. The concentrations of each natriuretic peptides were as follows: control (0 M) (white circle), $1 \times 10^{-9}$ M (black circle), $1 \times 10^{-8}$ M (white triangle), $1 \times 10^{-7}$ M (white square), and $1 \times 10^{-6}$ M (down-pointing black triangle) . The sample size at each point of the graph is n = 10 to 12.

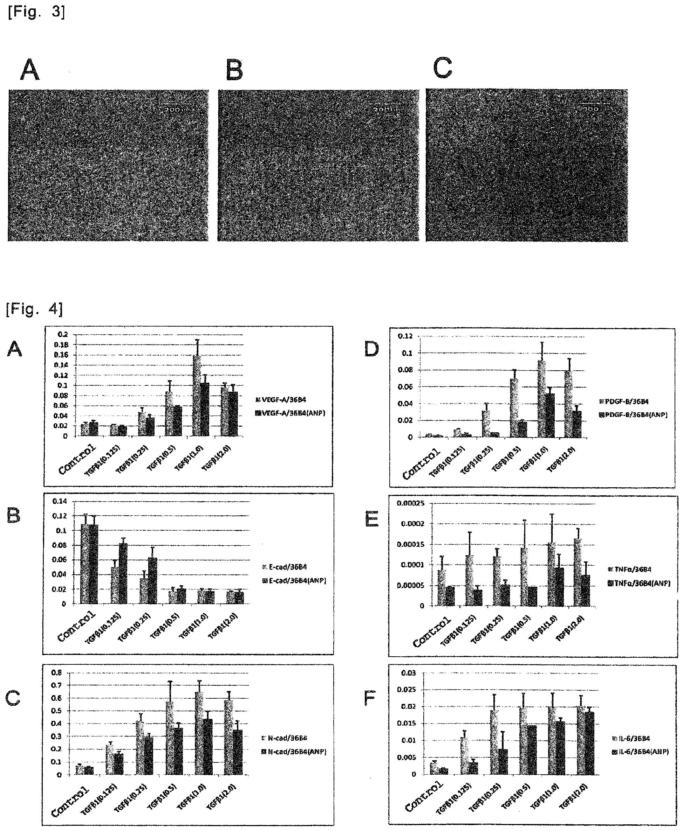

Fig. 3 shows micrographs showing the morphology change of A549 cells responding to stimulation by TGF-$\beta$1 in the presence (Fig. 3C) or absence (Fig. 3B) of 1 $\mu$M hANP (Fig. 3A: non-stimulated A549 cells (control), Fig. 3B: TGF-$\beta$1 (1 ng/mL), Fig. 3C: hANP (1 $\mu$M) + TGF-$\beta$1 (1 ng/mL)).

Fig. 4 shows graphs showing the gene expression of each factor (Fig. 4A: VEGF-A, Fig. 4B: E-cadherin, Fig. 4C: N-cadherin, Fig. 4D: PDGF-B, Fig. 4E: TNF-$\alpha$, and Fig. 4F: IL-6) as an indicator of EMT based on the mRNA level responding to stimulation of A549 cells by 0.25, 0.5, and 1.0 ng/mL of TGF-$\beta$1 in the presence or absence of 1 $\mu$M hANP. In each graph, the horizontal axis indicates TGF-$\beta$1 concentration and the vertical axis indicates mRNA concentration. In each graph, the gene expression in the absence of 1 $\mu$M hANP is shown by gray bars, and the gene expression in the presence of 1 $\mu$M hANP is shown by black bars.

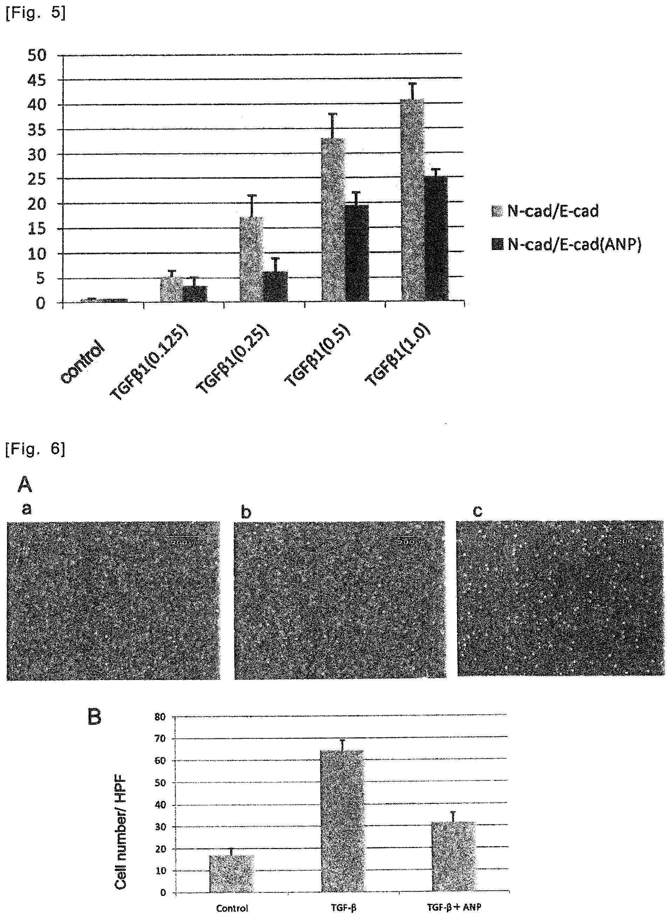

Fig. 5 shows a graph showing the expression ratio of N-Cadherin to E-Cadherin (N-Cad/E-Cad) responding to stimulation of A549 cells by 0.25, 0.5, and 1.0 ng/mL of TGF-$\beta$1 in the presence or absence of 1 $\mu$M hANP. The horizontal axis indicates TGF-$\beta$1 concentration and the vertical axis indicates the expression ratio of N-Cadherin to E-Cadherin (N-Cad/E-Cad) . The expression ratio of N-Cadherin to E-Cadherin (N-Cad/E-Cad) in the absence of 1 $\mu$M hANP is shown by gray bars, and the expression ratio of N-Cadherin to E-Cadherin (N-Cad/E-Cad) in the presence of 1 $\mu$M hANP is shown by black bars.

Fig. 6A and Fig. 6B show the migration ability of A549 cells in the presence or absence of hANP in the Boyden Chamber Assay using TGF-$\beta$1 as a migration inducing substance. Fig. 6A shows micrographs of the cells on the lower surface of the cell culture insert 20 hours after the assay for each group (Fig. 6Aa : control group, Fig. 6Ab: TGF-$\beta$1 group, Fig. 6Ac: TGF-$\beta$1 + hANP group) . Fig. 6B shows a graph showing the number of the cells adhering to the lower surface of the culture insert 20 hours after the assay for each group.

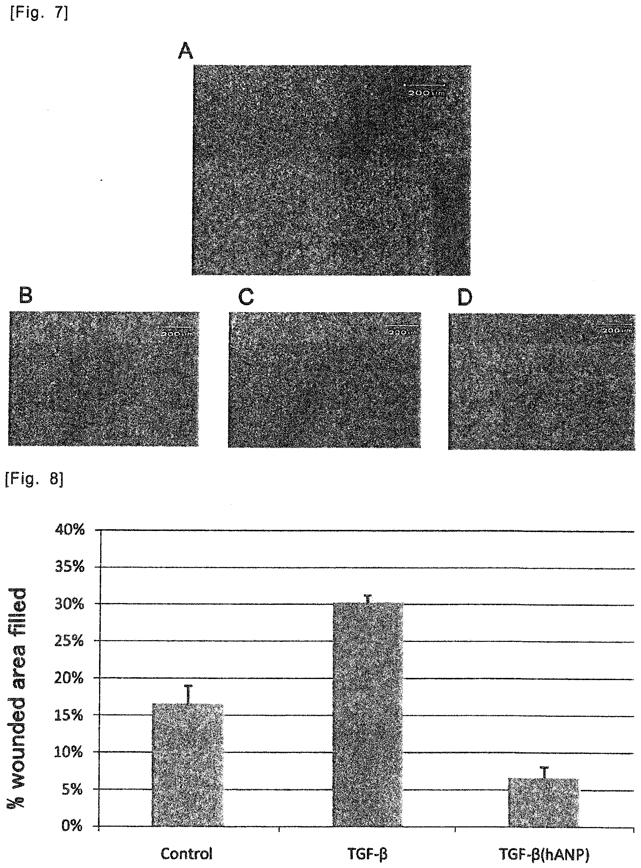

Fig. 7 shows a micrograph of a wounded area on a dish immediately after the wounded area was created (Fig. 7A) in the Wound Healing Assay and micrographs of wounded areas on dishes (Fig. 7B: control group, Fig. 7C: TGF-$\beta$1 group, and Fig. 7D: TGF-$\beta$1 + hANP group) 24 hours after the wounded areas were created.

Fig. 8 shows a graph showing the % wounded area filled of the control group, the TGF-$\beta$1 group, and the TGF-$\beta$1 + hANP group 24 hours after the wounded areas were created in the Wound Healing Assay.

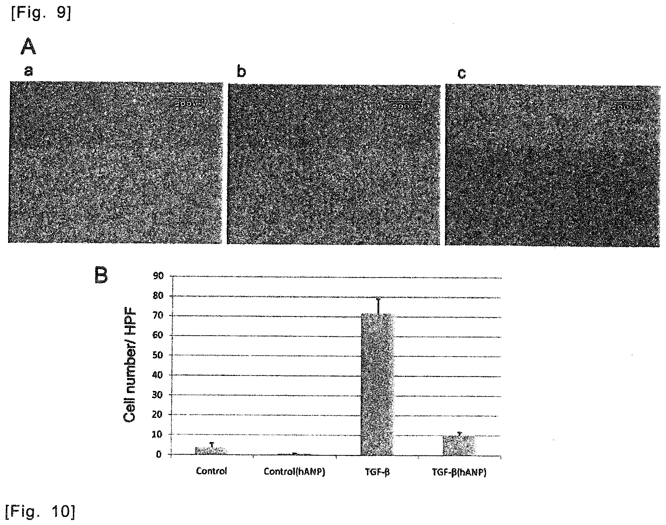

Fig. 9A and Fig. 9B show the invasion ability of A549 cells in the presence or absence of hANP in the Boyden Chamber Assay in which TGF-$\beta$1 as a migration inducing substance was used and a Matrigel layer was formed on the upper surface. Fig. 9A shows micrographs of the lower surface of the cell culture insert 40 hours after the assay for each group (Fig. 9Aa : control group, Fig. 9Ab: TGF-$\beta$1 group, Fig. 9Ac: TGF-$\beta$1 + hANP group) . Fig. 9B shows the graph for each group which shows the number of the cells adhering to the lower surface of the cell culture insert 40 hours after the assay.

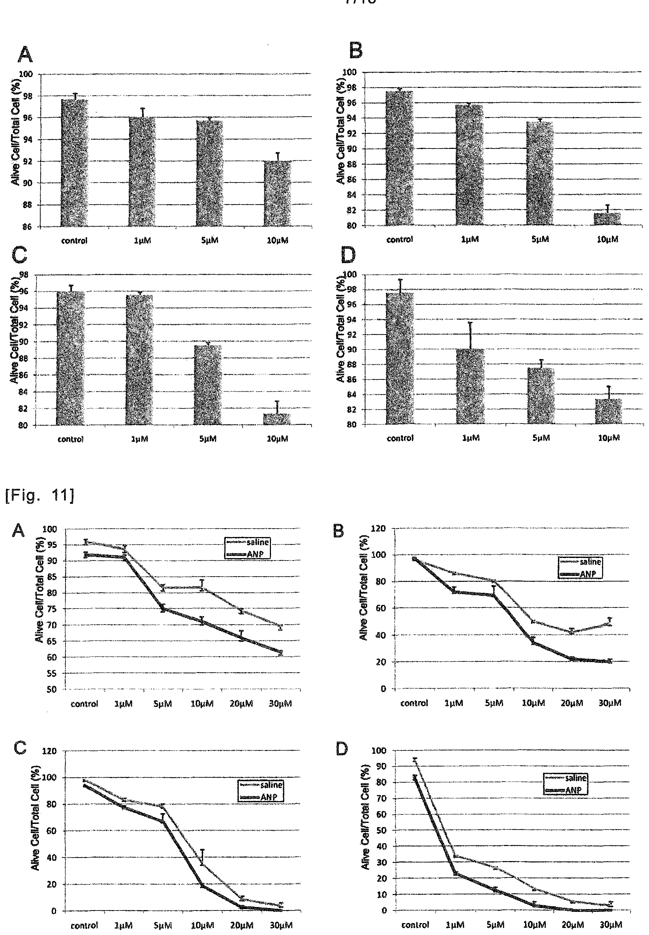

Fig. 10 shows graphs showing apoptosis induction in various cancer cells (Fig. 10 A: A549 cells, Fig. 10B: H460 cells, Fig. 10C: H520 cells, and Fig. 10D: H358 cells) in a hANP-concentration-dependent manner (1, 5, and 10 $\mu$M) .

Fig. 11 shows graphs showing apoptosis induction by 1 $\mu$M hANP in various cancer cells (Fig. 11 A: A549 cells, Fig. 11B: H460 cells, Fig. 11C: H520 cells, and Fig. 11D: H358 cells) at each concentration of cisplatin. In each graph, the horizontal axis indicates the cisplatin concentration. In each graph, the gray line is for the cisplatin (CDDP) alone group and the black line is for the 1 $\mu$M hANP group.

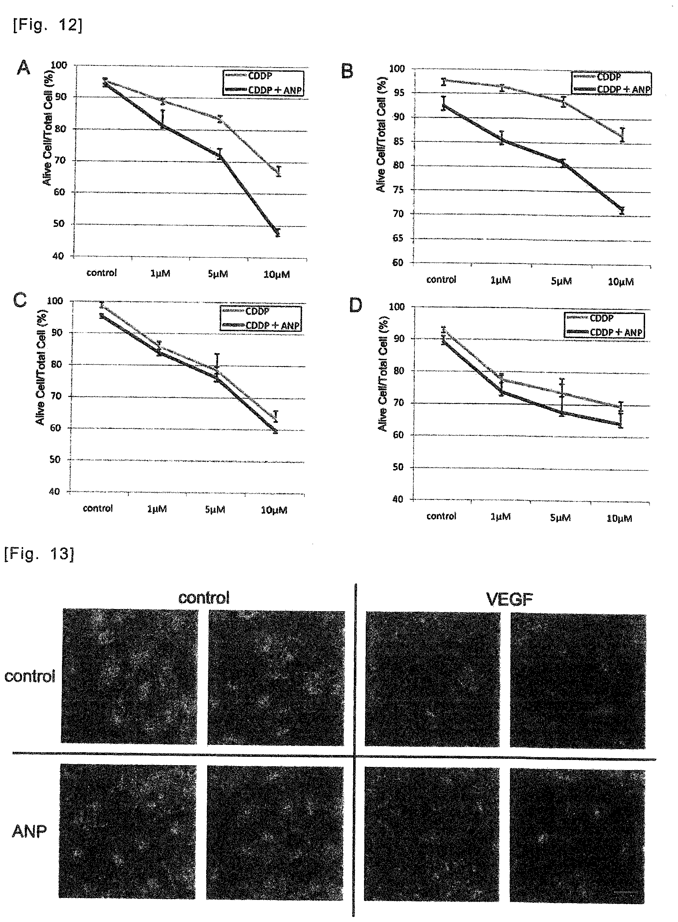

Fig. 12 shows graphs showing apoptosis induction by 10 $\mu$M hANP in various cancer cells (Fig. 12A: A549 cells, Fig. 12B: H460 cells, Fig. 12C: H520 cells, and Fig. 12D: H358 cells) at each concentration of cisplatin. In each graph, the horizontal axis indicates the cisplatin concentration. In each graph, the gray line is for the cisplatin (CDDP) alone group and the black line is for the 10 $\mu$M hANP group.

Fig. 13 shows micrographs showing the effects of VEGF and hANP on the intercellular adhesion of confluently cultured HUVEC cells (upper left: control, upper right: HUVEC cells with added VEGF, lower left: HUVEC cells with added hANP, lower right: HUVEC cells with added VEGF and hANP) . In Fig. 13, parts stained in a circle or an ellipse (shown in gray (in blue in the actual color micrographs)) are cellular nuclei stained with Topro3. The belt-like stained parts close to the outer edges of the cells (belt-like stained parts other than the nuclei: in gray (in green in the actual color micrographs)) are immunostained VE-cadherin and show the state of intercellular adhesion.

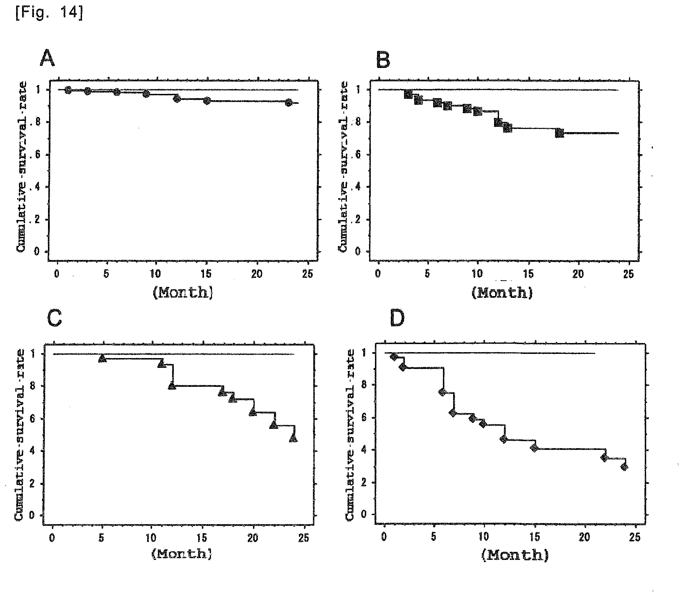

Fig. 14 shows graphs showing the recurrence-free survival rate (cumulative survival rate) of the hANP group and the control group analyzed by the Kaplan-Meier method (censor variable: cancer recurrence) in an early postsurgical period (up to 2 years) of the patients after removal of lung cancer. Each graph is for different cancer stages (Fig. 14A: Stage IA, Fig. 14B: Stage IB, Fig. 14C: Stage II, Fig. 14D: Stage III) . The average observation periods of the hANP group and the control group were 21.8 months and 22.0 months, respectively. In Fig. 14A, Fig. 14B, Fig. 14C, and Fig. 14D, black circles, black squares, black triangles, and black diamonds each represent the values of the control group. In each graph, the absence of plotted points means that there was no event, such as death or recurrence.

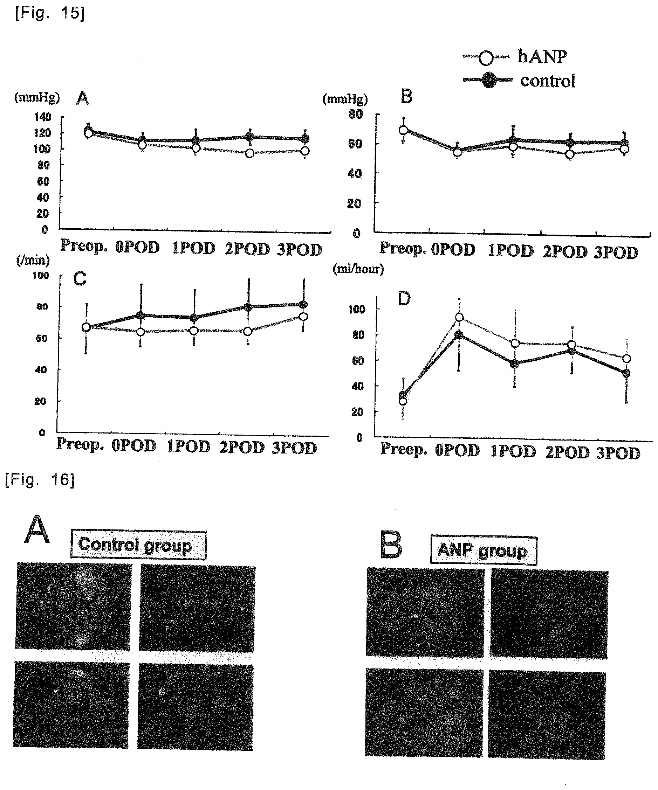

Fig. 15 shows graphs showing the diastolic blood pressure (Fig. 15A), the systolic blood pressure (Fig. 15B), the heart rate (Fig. 15C), and the urine volume (Fig. 15D) of the hANP group (white circle) (n = 23) and the control group (black circle) (n = 21) from before the cancer removal to day 4 after the surgery.

Fig. 16 shows fluorescence micrographs of the lung at 8 weeks from the injection of fluorescently labeled A549 tumor cells into the tail vein of mice in a tail vein injection metastasis test. Fig. 16A shows photographs of the control group (physiological saline was administered for 28 days), and Fig. 16B shows photographs of the ANP group was administered at 0.5 $\mu$g/kg/min for 28 days).

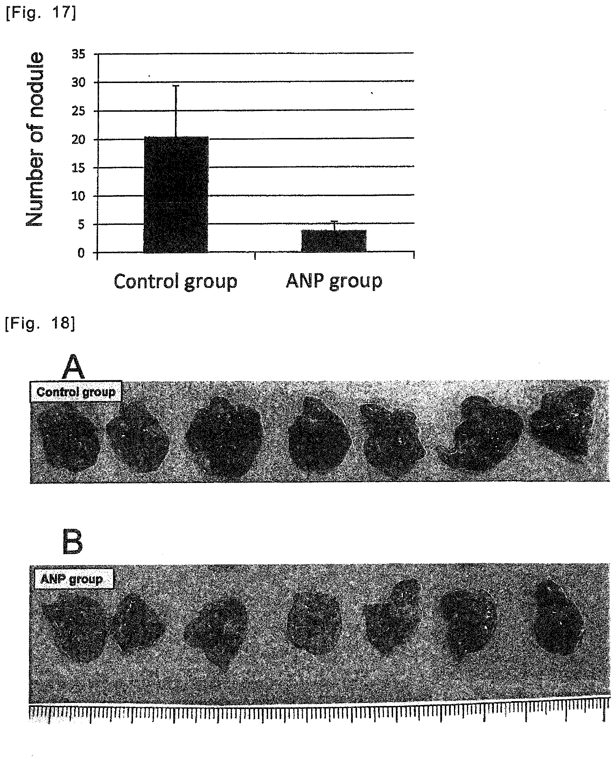

Fig. 17 shows a graph showing the number of nodules formed due to lung metastasis at 8 weeks from the injection of GFP-labeled A549 tumor cells into the tail vein of mice in a tail vein injection metastasis test. The vertical axis indicates the number of nodules formed in the lung. The left bar is for the control group (physiological saline was administered for 28 days), and the right bar is for the ANP group was administered at 0.5 $\mu$g/kg/min for 28 days).

Fig. 18 shows micrographs of the lung at 2 weeks from the injection of mouse melanoma B16-F10 tumor cells into the tail vein of mice in a tail vein injection metastasis test. Black parts are nodules (metastatic foci) formed by metastasized melanoma. Fig. 18A shows a photograph of the control group (physiological saline), and Fig. 18B shows a photograph of the ANP group (hANP: 0.5 $\mu$g/kg/min).

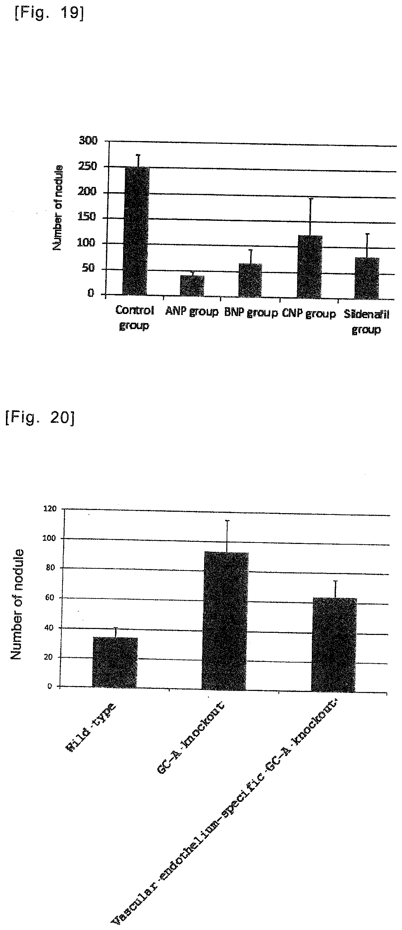

Fig. 19 shows a graph showing the number of nodules formed due to lung metastasis at 2 weeks from the injection of mouse melanoma B16-F10 tumor cells into the tail vein of mice in a tail vein injection metastasis test. The vertical axis indicates the number of nodules formed in the lung per individual. Each bar is for, from the left to the right, the control group (physiological saline), the ANP group (hANP: 0.5 $\mu$g/kg/min, continuous administration), the BNP group (mouse BNP: 0.5 $\mu$g/kg/min, continuous administration), the CNP group (hCNP-22: 2.5 $\mu$g/kg/min, continuous administration), and the sildenafil group (sildenafil citrate: 20 mg/kg, intraperitoneal administration).

Fig. 20 shows a graph showing the number of nodules formed due to lung metastasis at 2 weeks from the injection of mouse melanoma B16-F10 tumor cells into the tail vein of GC-A knockout mice in a tail vein injection metastasis test. The vertical axis indicates the number of nodules formed in the lung per individual. The left bar is for wild type mice, the center bar is for whole body GC-A knockout mice, and the right bar is for endothelial cell-specific GC-A knockout mice.

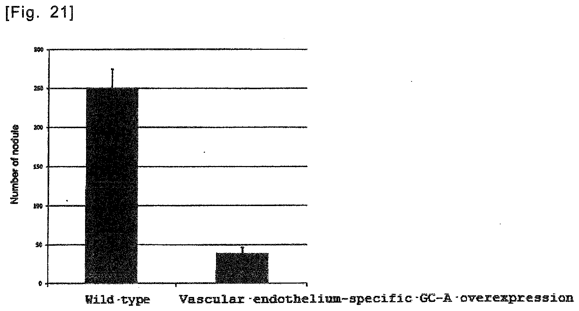

Fig. 21 shows a graph showing the number of nodules formed due to lung metastasis at 2 weeks from the injection of mouse melanoma B16-F10 tumor cells into the tail vein of endothelial cell-specific GC-A overexpression mice in a tail vein injection test. The vertical axis indicates the number of nodules formed in the lung per individual. The right bar is for vascular endothelial cell-specific GC-A overexpression mice, and the left bar is for wild type mice.

Description of Embodiments

Vascular endothelial intracellular cGMP enhancer

In the present invention, the vascular endothelial intracellular cGMP enhancer is a medicinal agent which at least has an activity of acting on the endothelial cells of a lymphatic vessel or a blood vessel in the living body to increase the intracellular cGMP concentration and resulting signaling (intracellular cGMP enhancing activity). Since the cGMP in vascular endothelial cells are also released into the blood, this enhancing activity is observable in the living body as an increase in the cGMP concentration not only in vascular endothelial cells but also in the blood. Intracellular cGMP is produced by guanylate cyclase (GC, which includes transmembrane receptor type, such as GC-A and GC-B, and soluble type (sGC)), and therefore activation of GC increases the cGMP concentration. In addition, the intracellular cGMP is degraded by phosphodiesterase 5 (PDE5), and therefore a PDE5 inhibitor, for example sildenafil, also increases the intracellular cGMP concentration. Further, addition of exogenous cGMP or an analog thereof can increase signaling which is mediated by the increase in the intracellular cGMP concentration. The intracellular cGMP enhancing activity in the present invention may be based on any mechanism as long as the activity directly or indirectly increases signaling via increase in intracellular cGMP concentration. Preferred mechanisms are activation of GC-A, activation of GC-B, and inhibition of PDE5, and more preferred is activation of GC-A.

As transmembrane receptor type GC, the natriuretic peptide receptor GC-A and the natriuretic peptide receptor GC-B have been confirmed to express in vascular endothelial cells. Therefore, agonists thereof, such as natriuretic peptides including ANP and CNP, can be used as the vascular endothelial intracellular cGMP enhancer of the present invention. In particular, GC-A agonists have, in addition to the action on vascular endothelium, activities which inhibit EMT, migration activity, etc. of tumor cells of epithelial malignant tumors where GC-A is expressed. For this reason, GC-A agonists exhibit a favorable metastasis suppressing effect on epithelial malignant tumors. Various natriuretic peptides are cleaved by neutral endopeptidase (NEP) and lose the activities thereof. Therefore, a NEP inhibitor can also be used as an intracellular cGMP enhancer of the present invention.

In addition, guanylin, uroguanylin, etc., which are agonists of GC-C known to have an action mainly exhibited in the digestive tract, are also known to have an intracellular cGMP enhancing activity.

As soluble type GC, sGCα, sGCβ, etc. are known, and these are activated by nitric oxide (NO). Therefore, NO donors, such as nitroglycerin; eNOS substrates (such as arginine) and activators of eNOS, which is a NO-producing enzymes expressed in endothelial cells can also be used as a vascular endothelial intracellular cGMP enhancer of the present invention.

Most of the metastasis of tumor cells is achieved by adhesion of the tumor cells to the endothelial tissue of a peripheral blood vessel or a peripheral lymphatic vessel. Therefore, the vascular endothelial intracellular cGMP enhancer of the present invention is preferably capable of increasing the endothelial intracellular cGMP concentration in a peripheral blood vessel or increasing the cGMP concentration in the blood when administered to a living body.

Regarding the vascular endothelial intracellular cGMP enhancer used for the present invention, the intracellular cGMP enhancing activity against the vascular endothelial cells can be confirmed by a conventional method based on the findings of the present invention. For example, a test substance is added to a culture of cells derived from the vascular endothelium, such as HUVEC or a culture of vascular endothelial tissue collected from an animal, and after a certain period of culture, the cGMP concentration in the cells or in the culture media is measured using a commercially available cGMP measuring kit (for example, the Cyclic GMP Assay Kit made by YAMASA Corp.). By comparing the measurement results with those in cases where the test substance is not added, the increase in the cGMP concentration can be detected. Alternatively, the intracellular cGMP enhancing activity against the vascular endothelial cells can be measured by administering the test substance to an experimental animal, such as a mouse, measuring the cGMP concentration in the blood, and then comparing the measurement results with the cGMP concentration in the blood of the same animal before the administration or the cGMP concentration in the blood of a non-administration group.

The vascular endothelial intracellular cGMP enhancer used for the present invention is not particularly limited as long as the enhancer has an activity of acting on vascular endothelial cells to increase the intracellular cGMP concentration, and examples thereof include a natriuretic peptide receptor GC-A agonist, a natriuretic peptide receptor GC-B agonist, a GC-C agonist, a NEP inhibitor, a PDE5 inhibitor, a NO donor, an eNOS activator, a cGMP analog, etc., and preferred are a natriuretic peptide receptor GC-A agonist, a natriuretic peptide receptor GC-B agonist, a NEP inhibitor, and a PDE5 inhibitor, and more preferred is a natriuretic peptide receptor GC-A agonist.

Natriuretic peptide receptor GC-A agonist

In the present invention, the "natriuretic peptide receptor GC-A agonist" means a substance which binds to the natriuretic peptide receptor GC-A (hereinafter, may be simply written as "GC-A" (Chinkers M, et al., Nature 338; 78-83, 1989)) and which then exhibits an action to activate guanylate cyclase (hereinafter, "GC-A agonist activity"), and such a substance may be simply written as "GC-A agonist" herein. Representative examples of the GC-A agonist include atrial natriuretic peptide (ANP) and brain natriuretic peptide (BNP). The GC-A agonist of the present invention is not particularly limited as long as it has a GC-A agonist activity, and ANP, BNP, an active fragment thereof, a mutant thereof, a derivative thereof, and a modified form thereof may be used. In addition, peptides and low-molecular compounds which have a GC-A agonist activity are included in the GC-A agonist of the present invention even if they do not have any structure in common with ANP or BNP.

Examples of the ANP in the present invention include a 28-amino acid ANP of human origin (SLRRSSCFGG RMDRIGAQSG LGCNSFRY: SEQ ID NO: 1) and a 28-amino acid ANP of rat origin (SLRRSSCFGG RIDRIGAQSG LGCNSFRY: SEQ ID NO: 2). As an ANP of human origin, the α-hANP described in Biochem. Biophys. Res. Commun., vol. 118, p. 131, 1984 has obtained marketing approval in Japan under a generic name of carperitide and is sold under a trade name of HANP. The α-ANP is also generally known as human pro-ANP [99-126].

Examples of the BNP in the present invention include a 32-amino acid BNP of human origin (SPKMVQGSGC FGRKMDRISS SSGLGCKVLR RH: SEQ ID NO: 3), a 32-amino acid BNP of porcine origin (SPKTMRDSGC FGRRLDRIGS LSGLGCNVLR RY: SEQ ID NO: 4), and a BNP of rat origin (SQDSAFRIQE RLRNSKMAHS SSCFGQKIDR IGAVSRLGCD GLRLF: SEQ ID NO: 5). The human BNP has obtained pharmaceutical approval in the US etc. under a generic name of nesiritide and is sold under a trade name of Natrecor.

The amino acid sequence Cys-Phe-Gly-Xaa1-Xaa2-Xaa3-Asp-Arg-Ile-Xaa5-Xaa6-Xaa7-Xaa8-Xaa9-Leu-Gly-Cys-Xaa10-Xaa11-Xaa12-Arg (wherein, Xaa3 is Met, Leu, or Ile) (SEQ ID NO: 6, hereinafter referred to as "ring structure sequence A") is well conserved among various ANPs and BNPs in the amino acid sequence consisting of a 17-amino acid ring formed by a disulfide bond between two cysteine residues in the sequence (for example, in hANP, the two Cys residues at position 7 and position 23 in SEQ ID NO: 1 bind to each other by disulfide bonding to form a ring structure, and in hBNP, the two Cys residues at position 10 and position 26 in SEQ ID NO: 3 bind to each other by disulfide bonding to form a ring structure) and the amino acid sequence following the C terminus of the ring and thus considered to be important for the binding to a receptor and for the expression of the activity (Silver MA, Curr. Opin. Nephrol. Hypertens., vol. 15, 14-21, 2006; and A. Calderone, Minerva Endocrinol., 29, 113-127, 2004).

Natriuretic peptide receptor GC-B agonist

In the present invention, the "natriuretic peptide receptor GC-B agonist" means a substance which binds to a natriuretic peptide receptor GC-B (hereinafter, may be simply written as "GC-B" (Suga S., et al., Endocrinology, 130 (1), 229-239, 1992)) to exhibit an action to activate guanylate cyclase (hereinafter, "GC-B agonist activity"), and such a substance may be simply written as "GC-B agonist" herein. Representative examples of the GC-B agonist include c-type natriuretic peptide (CNP). The GC-B agonist of the present invention is not particularly limited as long as it has a GC-B agonist activity, and CNP, an active fragment thereof, a mutant thereof, a derivative thereof, and a modified form thereof may be used. In addition, peptides and low-molecular compounds which have a GC-B agonist activity are included in the agonist of the present invention even if they do not have any structure in common with CNP.

Examples of the CNP in the present invention include a 22-amino acid CNP-22 of human origin (GLSKGCFGLK LDRIGSMSGL GC: SEQ ID NO: 7, herein also referred to as hCNP-22 and mammals, such as a porcine, a rat, etc. have this amino acid sequence in common), a 53-amino acid CNP-53 of human origin (DLRVDTKSRA AWARLLQEHP NARKYKGANK KGLSKGCFGL KLDRIGSMSG LGC: SEQ ID NO: 8, herein also referred to as hCNP-53), a CNP of fowl origin (GLSRSCFGVK LDRIGSMSGL GC: SEQ ID NO: 9) and a CNP of frog origin (GYSRGCFGVK LDRIGAFSGL GC: SEQ ID NO: 10).

In various CNPs, the ring structure formed by a disulfide bond between two cysteine residues in the sequence (for example, in hCNP-22, the two Cys residues at position 6 and position 22 in SEQ ID NO: 7 bind to each other by disulfide bonding to form a ring structure) is considered to be important for the binding to the GC-B receptor and for the expression of the activity (Furuya, M. et al., Biochem. Biophys. Res. Commun., 183 (3), 964-969, 1992; Silver MA, Curr. Opin. Nephrol. Hypertens., vol. 15, 14-21, 2006; and A. Calderone, Minerva Endocrinol., 29, 113-127, 2004). Furuya et al. reported that hCNP6-22, which is a peptide consisting of only the ring structure (a peptide consisting of amino acids from position 6 to position 22 of SEQ ID NO: 7), and the ring structure in which the N-terminal and C-terminal sequences of ANP are added to the N-terminus and the C-terminus exhibit a GC-B agonist activity comparable to that of hCNP-22. Furuya et al. also reported that the hCNP-22 having mutations at position 9 (Leu) and position 10 (Lys) has a reduced activity but the peptide having mutations at other positions (for example, at position 17 (Ser) and position 18 (Met)) and a peptide obtained by replacing the amino acid sequence from position 10 to position 12 of hANP with the corresponding sequence of hCNP-22, Leu-Lys-Leu (from position 9 to position 11 of SEQ ID NO: 7) exhibit a GC-B agonist activity comparable to that of hCNP-22. Based on the findings, the amino acid sequence of the ring structure important for the GC-B agonist activity is Cys-Phe-Gly-Leu-Lys-Leu-Asp-Arg-Ile-Gly-Xaa1-Xaa2-Ser-Gly-L eu-Gly-Cys (wherein, Xaa1 is Ser or Ala, and Xaa2 is Met, Phe or Glu: SEQ ID NO: 11), which is hereinafter referred to as "ring structure sequence B".

In the present invention, the "active fragment" of a biologically active peptide or protein means a fragment which consists of the biological activity-related site of the peptide or protein and which retains at least part of the biological activity of the peptide or protein. As an active fragment of the ANP or BNP of the present invention, a peptide which binds to the above-described GC-A and activates guanylate cyclase can be used. Examples of such an active fragment include a peptide comprising the ring structure A (the amino acid sequence of SEQ ID NO: 6), and preferred is a peptide consisting of the ring structure A (the amino acid sequence of SEQ ID NO: 6). Specific examples thereof include a peptide consisting of the amino acid sequence from position 7 to position 27 of SEQ ID NO: 1, the amino acid sequence from position 10 to position 30 of SEQ ID NO: 3 or 4, or the amino acid sequence from position 23 to position 43 of SEQ ID NO: 5, but are not limited thereto. Any fragment which has the ring structure A and has a GC-A agonist activity can be used as the active fragment of the ANP or BNP of the present invention.

As an active fragment of the CNP of the present invention, for example, a peptide which consists of at least part of any of SEQ ID NOs: 7 to 10 and has a GC-B agonist activity can be used. Examples of such an active fragment include a peptide comprising the ring structure B (the amino acid sequence of SEQ ID NO: 11), and preferred is a peptide consisting of the ring structure B (the amino acid sequence of SEQ ID NO: 11). Specific examples thereof include hCNP6-22, a peptide consisting of the amino acid sequence of SEQ ID NO: 7, 9, or 10 in which contiguous amino acids from position 1 to position 5 is partially or completely deleted (i.e., partial or complete deletion of the 1st to the 5th sequence), and a peptide consisting of the amino acid sequence of SEQ ID NO: 8 in which contiguous amino acids from position 1 to position 36 is partially or completely deleted (i.e., partial or complete deletion of the 1st to the 36th sequence), but are not limited thereto. Any fragment which has the ring structure B and has a GC-B agonist activity can be used as the active fragment of the CNP of the present invention.

The GC-A agonist and the GC-B agonist of the present invention may be the above-described active fragment itself, or a peptide in which one or more amino acids are added to the N-terminus, the C-terminus, or both termini of the active fragment (a derivative of an active fragment) as long as the peptide retains the desired agonist activity. Examples of the peptide include a peptide in which a sequence derived from the N-terminal of ANP and/or a sequence derived from the C-terminus of ANP is added to the N-terminus and/or the C-terminus of hCNP6-22 (Furuya, M. et al., Biochem. Biophys. Res. Commun., 183 (3), 964-969, 1992).

In the present invention, the "mutant" of a biologically active peptide or protein means a peptide which has substitution, deletion, insertion and/or addition (hereinafter referred to as "substitution etc.") of one to several amino acids at one to several positions of the amino acid sequence but retains at least part of the biological activity of the original peptide or protein. "Several positions" means usually about 3 positions and preferably about 2 positions. "Several amino acids" means usually about 10 amino acids, preferably about 5 amino acids, more preferably about 3 amino acids, and still more preferably about 2 amino acids. In cases where substitution etc. occurs at two or more positions, the substitution etc. may be any one of substitution, deletion, insertion and addition, or two or more thereof in combination. The amino acid which is subjected to substitution etc. may be a naturally-occurring amino acid, a modified form thereof such as an acylated form, or an artificially synthesized amino acid analog. The position of substitution etc. may be any position as long as part of the activity of the original peptide or protein is retained, but preferred is a position other than a position involved in the active site or the receptor binding site of the original peptide or protein. As a mutant of ANP or BNP, any mutant having substitution etc. at any desired position may be used as long as the GC-A agonist activity is retained. However, preferred examples thereof include a peptide in which the ring structure A mentioned above is retained and substitution etc. occurs outside the ring structure.

For example, a mutant of ANP may have substitution etc. of one to several amino acids at any one to several desired positions in SEQ ID NO: 1 or 2 as long as the GC-A agonist activity is retained. However, a preferred mutant has substitution etc. of one to several amino acids at one to several positions other than the amino acids shown in SEQ ID NO: 6 which is included in SEQ ID NO: 1 or 2, and a more preferred mutant has substitution etc. of one to several amino acids at one to several positions of positions 1 to 6 and 28 in SEQ ID NO: 1 or 2. Also, a mutant of BNP may have substitution etc. of one to several amino acids at any one to several desired positions in SEQ ID NO: 3, 4, or 5 as long as the GC-A agonist activity is retained. However, a preferred mutant has substitution etc. of one to several amino acids at one to several positions other than the amino acids shown in SEQ ID NO: 6 which is included in SEQ ID NO: 3, 4, or 5. More preferred is a peptide having substitution etc. of one to several amino acids at one to several positions of positions 1 to 9, 31, and 32 in SEQ ID NO: 3 or 4, or a peptide having substitution etc. of one to several amino acids at one to several positions of positions 1 to 22, 44, and 45 in SEQ ID NO: 5.

Specific examples of the mutant of ANP include rat $\alpha$-rANP identical to a hANP having substitution of Ile for Met at position 12 (Biochem. Biophys. Res. Commun., vol. 121, p. 585, 1984), and a hANP having deletion of Ser-Leu-Arg-Arg-Ser-Ser at the N-terminus. Examples of such mutants of ANP or BNP include a series of peptides described in Medicinal Research Review, vol. 10, p. 115, 1990. Examples of a mutant having deletion of one to several amino acids and substitution of one to several amino acids by other amino acids include mini-ANP consisting of 15 amino acid residues (Science, vol. 270, p. 1657, 1995) .

As a mutant of CNP, any mutant having substitution etc. at any desired position may be used as long as the GC-B agonist activity is retained. However, preferred examples thereof include a peptide in which the ring structure B mentioned above is retained and substitution etc. occurs outside the ring structure. A specific mutant of CNP may have substitution etc. of one to several amino acids at any one to several desired positions in any of SEQ ID NOs: 7 to 10 as long as the GC-B agonist activity is retained. However, a preferred mutant has substitution etc. of one to several amino acids at one to several positions other than the amino acids shown in SEQ ID NO: 11 which is included in any of SEQ ID NOs: 7 to 10. More preferred is a mutant having substitution etc. of one to several amino acids at one to several positions of positions 1 to 5 in SEQ ID NO: 7, 9, or 10, or a mutant having substitution etc. of one to several amino acids at one to several positions of positions 1 to 36 in SEQ ID NO: 8.

Reported as specific examples of mutants of CNP are as follows: a peptide identical to hCNP-22 having mutation at positions 17 and 18 exhibits a GC-B agonist activity comparable to that of hCNP-22; even a derivative of the mutant having substitution of the N-terminus and the C-terminus of the ring structure by a sequence derived from hANP exhibits about 90% of the GC-B agonist activity of hCNP-22; a peptide identical to hCNP-22 having a mutation at any one position of positions 9 to 11 exhibits 50% or more of the GC-B agonist activity of hCNP-22; a peptide identical to hCNP-22 having mutation at both of positions 10 and 11 exhibits 40% or more of the GC-B agonist activity of hCNP-22; etc. (Furuya, M. et al., Biochem. Biophys. Res. Commun., 183 (3), 964-969, 1992). Another reference describes that various mutants of hCNP-22 retain the GC-B agonist activity and further exhibit resistance to cleavage by neutral endopeptidase (NEP), which is a degrading enzyme of CNP (WO 2009/067639). CD-NP is known as another derivative of hCNP-22. CD-NP is a peptide in which the C-terminal sequence of dendroaspis natriuretic peptide (DNP: a natriuretic peptide derived from snake poison) is added to the C-terminus of hCNP-22 and is known to have both the GC-A agonist activity and the GC-B agonist activity (Deborah et al., J. Biol. Chem., vol. 289, No. 50, 35003-35009, 2008).

In the present invention, the "derivative" of a biologically active peptide or protein means a fusion peptide to which an additional peptide or protein is added and which comprises the amino acid sequence of the original peptide or protein and retains at least part of the biological activity of the original physiologically active peptide or protein. A fusion peptide having at least part of such a biological activity (in the present invention, an action of binding to GC-A or GC-B to activate guanylate cyclase) is also referred to as a derivative of a physiologically active peptide. In the derivative of the present invention, the additional peptide may be fused to one or both of the C-terminus and the N-terminus of the original physiologically active peptide or protein. The additional peptide is not particularly limited, but preferred is a peptide not having physiological activity in itself. The additional peptide may be directly linked or linked via a linker sequence. Various linker sequences are known, but preferred are those containing an abundance of Gly, Ser, etc. Examples of the additional peptide include the Fc region of an immunoglobulin (preferably IgG), a serum albumin, and the C-terminal sequence of ghrelin (for example, a fusion protein in which ANP is linked to the Fc region of an immunoglobulin (see US2010-0310561 A etc.) and a fusion protein in which GLP-1 is linked to serum albumin (see WO2002/046227 etc.)). Examples of the derivative used as the GC-A agonist of the present invention include derivatives of ANP or BNP, derivatives of the active fragments of ANP or BNP, and derivatives of the mutants of ANP or BNP, and preferred are derivatives of hANP and derivatives of hBNP. Examples of the derivative used as the GC-B agonist of the present invention include derivatives of CNP, derivatives of the active fragments of CNP, and derivatives of the mutants of CNP, and preferred are derivatives of hCNP-22, derivatives of hCNP-53, and derivatives of hCNP6-22.

Examples of the derivative used as the GC-A agonist include a fusion protein in which ANP is linked to the Fc region of an immunoglobulin (see US2010-0310561 A etc.). This fusion protein is known to retain the biological activity of ANP and improve the retention in the blood. Examples of the derivatives of ANP and BNP include a series of peptides described in Medicinal Research Review, vol. 10, p. 115, 1990.

Specific examples of the derivatives of ANP and CNP include various ANP derivatives and CNP derivatives disclosed in WO2009/142307 (corresponding to US2010-305031 A). In the reference, it is reported that a derivative in which the C-terminus of ghrelin was added to a physiologically active peptide, such as ANP, CNP, and motilin, retained the biological activity of the original peptide and improved the retention in the blood. It is also reported that all of the various derivatives in which a partial peptide derived from the C-terminus of ghrelin was added to one or both of the C-terminus and the N-terminus of hANP retained the GC-A agonist activity and their half-lives in the blood were extended. Also, all of the various derivatives in which a peptide sequence or peptide fragment derived from the C-terminus of ghrelin was added to one or both of the C-terminus and the N-terminus of hCNP6-22 retained the GC-B agonist activity and their half-lives in the blood were extended.

Further, a peptide in which the C-terminal part of ANP is added to the C-terminus of hCNP-22 and a peptide in which the N-terminal part and the C-terminal part of ANP are added to the N-terminus and the C-terminus of hCNP6-22 (derivatives of active fragments of CNP), which are other examples of the derivatives of CNP or the derivatives of the active fragments of CNP, retained the GC-B agonist activity comparable to that of CNP-22. (Furuya, M. et al., Biochem. Biophys. Res. Commun., 183 (3), 964-969, 1992). Further, another reference (WO 2009/067639) describes that various derivatives of hCNP-22 and hCNP-53 retain the GC-B agonist activity, and that some of the derivatives have also resistance to degradation by NEP.

These known derivatives of the natriuretic peptides can be used as the GC-A agonist and/or the GC-B agonist of the present invention.

In the present invention, the "modified form" of a biologically active peptide or protein means a peptide or protein in which amino acids at one to several positions are modified as a result of chemical reaction with another chemical substance and which retains at least part of the biological activity of the peptide or protein. The position to be modified may be any position as long as the activity of the original peptide or protein is retained. In cases where modification is performed by adding a chemical substance which is large to some extent, for example a polymer, the modification preferably occurs at a position other than a position involved in the active site or the receptor binding site of the original peptide or protein. In cases where the modification is for prevention of cleavage by a degrading enzyme, a peptide or protein in which the site to be cleaved is modified can be used.

Various methods for chemical modification are known, and example thereof include addition of a polymer which can be used in pharmaceutical technology (pharmacologically usable polymer), such as polyethylene glycol (PEG) and polyvinyl alcohol (PVA), and addition of a compound as a linker to a side-chain amino group of a Lys residue etc. for binding to another protein etc. (for example, serum albumin) via the linker. However, the method for chemical modification is not limited thereto, and various methods can be used. The modified forms of various physiologically active peptides can be produced as needed referring to, for example, US2009-0175821 A etc. Preferred modified forms are prepared by chemical modification by addition of a pharmaceutically usable polymer.

For example, a modified form of ANP may have modification at any one to several desired positions in SEQ ID NO: 1 or 2 as long as the GC-A agonist activity is retained. However, a preferred modified form comprises the amino acid sequence of SEQ ID NO: 1 or 2 and has chemical modification of at least one of amino acids other than the amino acids shown in SEQ ID NO: 6. A more preferred modified form has modification at one to several position other than the amino acids shown in SEQ ID NO: 6 which is included in SEQ ID NO: 1 or 2, and a further more preferred modified form has modification at one to several position of positions 1 to 6 and 28 in SEQ ID NO: 1 or 2. A mutant of BNP may be modified at any one to several desired position in SEQ ID NO: 3, 4, or 5 as long as the GC-A agonist activity is retained. However, a preferred modified form comprises the amino acid sequence of SEQ ID NO: 3, 4, or 5 and has chemical modification of at least one of amino acids other than the amino acids shown in SEQ ID NO: 6. A more preferred modified form has modification at one to several position other than the amino acids shown in SEQ ID NO: 6 which is included in SEQ ID NO: 3, 4, or 5, and a further more preferred modified form has modification at one to several position of positions 1 to 9, 31, and 32 in SEQ ID NO: 3 or 4; or of positions 1 to 22, 44 and 45 in SEQ ID NO: 5. Further, the modified forms of the above-mentioned active fragments and mutants of ANP or BNP, and derivatives thereof are all included in the present invention. Such various modified forms may be used in the present invention as long as the modified forms retain the GC-A agonist activity.

Specific examples of the modified form which may be used as a GC-A agonist include various modified forms, for example, one in which a hydrophilic polymer, such as PEG and PVA or a hydrophobic group represented by a hydrocarbon group, such as an alkyl group and an aryl group is bound to hBNP, or a mutant or an active fragment thereof, and it is known that such modified forms retain the GC-A agonist activity (see US patent No. 7,662,773 etc.).

In the binding of ANP or BNP to the GC-A receptor, important parts are the ring structure and the C-terminal tail part of ANP and BNP. Therefore, even another sequence or substance is linked to the N-terminus of ANP or BNP, the added peptide or modifying substance hardly exerts influence on the ring structure, that is, does not inhibit the binding. For this reason, the GC-A agonist activity is retained in such a derivative or a modified form. This fact is proved by the above-mentioned large number of references.

A modified form of CNP may have modification at any one to several desired positions in any of SEQ ID NOs: 7 to 10 as long as the GC-B agonist activity is retained. However, a preferred modified form has modification at one to several positions other than the amino acids shown in SEQ ID NO: 11 which is included in any of SEQ ID NOs: 7 to 10. More preferred is a modified form having modification at one to several positions of positions 1 to 5 in SEQ ID NO: 7, 9, or 10. Since it is known that the cleavage occurs between Cys at position 1 and Phe at position 2 of SEQ ID NO: 11 in the ring structure B of various CNP peptides, in a modification for providing resistance to cleavage by neutral endopeptidase (NEP), this bond may be cleaved.

Further, the modified forms of the above-mentioned active fragments and mutants of CNP, and derivatives thereof are all included in the present invention. Such various modified forms may be used in the present invention as long as the modified forms retain the GC-B agonist activity.

Regarding specific examples of such CNP, an active fragment thereof, a mutant thereof, a derivative thereof, and a modified form thereof, for example, WO 2009/067639 discloses that a plurality of modified forms in which various hydrophilic polymers, such as PEG, are bound to hCNP-22 or hCNP-53, and of modified forms in which the peptide bond between Cys6 and Phe7 of hCNP-22 corresponding to the cleavage site by NEP is replaced by a pseudopeptide, such as ($-CH_2-NH-$) and ($-C(=O)-N(R)-$) (wherein R represents a lower alkyl group including methyl, ethyl, propyl, isopropyl, n-butyl, isobutyl, sec-butyl, and tert-butyl) retain the GC-B agonist activity and that many of them have better retention in the blood than hCNP-22. The modified forms of various physiologically active peptides can be produced as needed referring to, for example, US2009-0175821 A etc.

In the binding of CNP to the GC-B receptor, important part is the ring structure of CNP. Therefore, even another sequence or substance is linked to the C-terminus and/or N-terminus of CNP, the added peptide or modifying substance hardly exerts influence on the ring structure, that is, does not inhibit the binding. For this reason, the GC-B agonist activity is retained in such a derivative or a modified form. This fact is proved by the above-mentioned large number of references.

The above-described ANP, BNP and CNP, an active fragment thereof, a mutant thereof, a derivative thereof, and a modified form thereof may be harvested from natural cells or tissue, produced by a genetic engineering procedure or a cellular engineering procedure, or chemically synthesized. Further, modification of the amino acid residues or partial deletion of the amino acid sequence may be performed by enzymatic or chemical treatment. Such production can be appropriately performed by a conventional method, according to the references mentioned herein.

As the GC-A agonist or the GC-B agonist of the present invention, a single substance having both the properties of a GC-A agonist and a GC-B agonist (herein, also referred to as "double-activity substance") may be used. The double-activity substance is not particularly limited as long as it retains both the GC-A agonist activity and the GC-B agonist activity. Examples of the substance include a substance having the ring structure A (SEQ ID NO: 6) and the ring structure B (SEQ ID NO: 11) in a single molecule (for example, a fusion peptide comprising both sequences, a modified form in which a peptide consisting of one of the sequences and a peptide consisting of the other sequence are linked via a linker compound), a substance comprising an amino acid sequence having combined properties of the ring structure A and of the ring structure B, etc. As a specific example of the double-activity substance, CD-NP is known. CD-NP is a peptide in which the C-terminal sequence of dendroaspis natriuretic peptide (DNP: a natriuretic peptide derived from snake poison) is added to the C-terminus of hCNP-22 and is known to have both the GC-A agonist activity and the GC-B agonist activity (Deborah et al., J. Biol. Chem., vol. 289, No. 50, 35003-35009, 2008). Another example is a peptide in which the amino acids at positions 9 to 11 of hANP (SEQ ID NO: 1) are replaced by Leu-Lys-Lue, and this peptide retained both the GC-A agonist activity and the GC-B agonist activity (Furuya, M. et al., Biochem. Biophys. Res. Commun., 183 (3), 964-969, 1992).

As the GC-A agonist and GC-B agonist of the present invention, those which are antibodies against the natriuretic peptide receptor GC-A or the natriuretic peptide receptor GC-B and which have an agonist activity for the respective receptor can be used.

In the present invention, an "antibody" is an antibody which specifically binds to a target antigen (GC-A or GC-B), or a fragment retaining the binding activity to the antigen (binding fragment, for example, Fab fragment etc.). Examples of the antibody of the present invention include an antiserum, a polyclonal antibody, a monoclonal antibody, a chimeric antibody, a single strand antibody, a human type antibody, a human antibody, and an active fragment of any of these. These antibodies can be produced by a conventional method using, as immunogen, the whole or the extracellular domain of an immunogenic receptor. For the humanized antibody, for example, WO 91/009968 etc. can be referred to, and for the human type antibody, for example, WO 92/03918, WO 94/25585, etc. can be referred to, in addition to various known techniques. The produced antibodies can be measured for the GC-A agonist activity or the GC-B agonist activity by the methods mentioned described later for selection of antibodies having a desired activity.

The anti-GC-A antibody used as the GC-A agonist of the present invention is an antibody which specifically binds to the natriuretic peptide receptor GC-A and which has the GC-A agonist activity, or a binding fragment of the antibody. Preferred is an anti-GC-A monoclonal antibody, which is a monoclonal antibody, and more preferred is a humanized anti-GC-A antibody or an anti-GC-A antibody of the human type.

The anti-GC-B antibody used as the GC-B agonist of the present invention is an antibody which specifically binds to the natriuretic peptide receptor GC-B and which has the GC-B agonist activity, or a binding fragment of the antibody. Preferred is an anti-GC-B monoclonal antibody, which is a monoclonal antibody, and more preferred is a humanized anti-GC-B antibody or an anti-GC-B antibody of the human type.

A person skilled in the art can readily perform measurement using a conventionally known method to determine whether a substance has the GC-A agonist activity. Specific procedure is as follows. To cultured cells in which GC-A (Chinkers M, et al., Nature, 338; 78-83, 1989) is forcibly expressed, the substance is added, and then the intracellular cGMP concentration is determined. "Retain part of the GC-A agonist activity" usually means that, when a GC-A agonist substance and ANP or BNP (in cases where the agonist substance is one prepared based on the sequence of ANP or BNP, the reference peptide) are measured in a parallel manner for the GC-A agonist activity in the same measurement system, the peak value of the cGMP concentration increasing activity of the GC-A agonist substance is at least about 10% or more of that of ANP or BNP. Preferably about 30% or more is retained, more preferably about 50% or more is retained, and further more preferably about 70% or more is retained.

A person skilled in the art can readily perform measurement using a conventionally known method to determine whether a substance has the GC-B agonist activity. Specific procedure is as follows. To cultured cells in which GC-B (Chinkers M, et al., Nature, 338; 78-83, 1989) is forcibly expressed, the substance is added, and then the intracellular cGMP concentration is determined. "Retain part of the GC-B agonist activity" usually means that, when a GC-B agonist substance and CNP are measured in a parallel manner for the GC-B agonist activity in the same measurement system, the peak value of the cGMP concentration increasing activity of the GC-B agonist substance is at least about 10% or more of that of CNP. Preferably about 30% or more is retained, more preferably about 50% or more is retained, and further more preferably about 70% or more is retained.

Regarding the GC-A agonist activity or the GC-B agonist activity of the present invention, a substance having long duration of the activities in a living body to which the substance is administered can be used for the present invention even if the increase at the peak in the above measurement is not very significant. For such evaluation, a test substance is administered to an animal, and the cGMP concentration in the blood is measured over time. The obtained data are plotted on a graph of which the X axis represents time and the Y axis represents the cGMP concentration, and the area under the curve (AUC) is compared to that of a control substance, such as ANP and CNP for evaluation. A test substance which is evaluated as "retaining the activity" usually has about 10% or more of the activity of the control substance, and preferably has about 30% or more, more preferably has about 50% or more, and further more preferably has about 70% or more.

In addition, a low molecular compound which can improve the cGMP productivity in such an evaluation system when added thereto can be used as a GC-A agonist or a GC-B agonist in the present invention even if the compound (for example, a low-molecular compound) does not have any structure in common with a natriuretic peptide.

Preferred as the GC-A agonist of the present invention is ANP, BNP, an active fragment thereof having the ring structure A, or a mutant thereof having substitution etc. outside the ring structure A, a derivative thereof, or a modified form thereof. More preferred is hANP, hBNP, a derivative thereof, or a modified form thereof, and further more preferred is hANP or hBNP.

Preferred as the GC-B agonist of the present invention is CNP, an active fragment thereof having the ring structure B, or a mutant thereof having substitution etc. outside the ring structure B, a derivative thereof, or a modified form thereof. More preferred is hCNP-22, hCNP-53, an active fragment thereof, namely hCNP6-22, a derivative thereof, or a modified form thereof, and further more preferred is hCNP-22, hCNP-53, or hCNP6-22.

NEP inhibitor

The NEP inhibitor used for the present invention is not particularly limited as long as it has an activity of inhibiting the above-mentioned cleavage in the naturally occurring type of natriuretic peptide by NEP, and such a NEP inhibitor is described in a large number of references (for example, Cuculi, E et al., Expert Opinn. Investig. Drugs, 20 (4), 457-463, 2011 and Matthew I. et al., Br. J. Clin. Pharmacol., 57 (1), 27-36, 2004) . To achieve the objective of the present invention, the NEP inhibitor may be an NEP-selective inhibitor. Preferred examples of the NEP inhibitor of the present invention include sampatrilat, sinorphan, omapatrilat, Gemopatrilat, Fasidotril, Mixanpril, Z-13752A, MDI-100240, etc. These medicinal agents can be produced and formulated according to the above-mentioned references (including references cited therein) and by publicly known techniques.

PDE5 inhibitor

The PDE5 inhibitor used for the present invention is not particularly limited as long as the substance has an activity of acting on vascular endothelial cells to inhibit the degradation of cGMP by a PDE5 enzyme, and various publicly known agents can be used (for example, see M. P. Govannoni, et al., Curr. Med. Chem., 17, 2564-2587, 2010). Preferred examples thereof include sildenafil, vardenafil, tadalafil, udenafil, mirodenafil, SLx-2101, lodenafil, lodenafil carbonate, exisulind, and derivatives thereof and pharmacologically acceptable salts thereof. More preferred are sildenafil, vardenafil, tadalafil, udenafil, mirodenafil, and pharmacologically acceptable salts thereof; further more preferred are sildenafil citrate, vardenafil hydrochloride, tadalafil, udenafil, mirodenafil; and most preferred is sildenafil citrate. These medicinal agents can be produced and formulated according to the above-mentioned references (including references cited therein) and by publicly known techniques.

GC-C agonist

The GC-C agonist used for the present invention is not particularly limited as long as it has an activity of binding to GC-C to increase the intracellular cGMP concentration, and various publicly known substances can be used (for example, Potter L. R., Pharmacology & Therapeutics, 130 (1), 71-82, 2011) . Examples of the GC-C agonist used for the present invention include heat-stable enterotoxin (ST) and relative substances thereof, peptides, such as guanylin and uroguanylin, active fragments thereof, mutants thereof, derivatives thereof, and modified forms thereof. Preferred is linaclotide, guanylin or uroguanylin. These medicinal agents can be produced and formulated according to the above-mentioned references (including references cited therein) and by publicly known techniques.

NO donor

The NO donor used for the present invention is a substance which itself has a NO structure and releases NO, or a decomposed product or metabolite thereof which has a NO structure and releases NO. Various NO donors are known in the art (see, for example, J. Clin. Hypertens., No.12, vol.8, suppl. 4, 40-52, 2006), and examples thereof include NO gas, nitrate, and sydnomine. Examples of the sydnomine include molsidomine and linsidomine, which is an active metabolite of molsidomine. These NO donors are described in, for example, R. P. Mason, et al., J. Clin Hypertens., 8 (Sppl. S12), 40-52, 2006), and can be produced and formulated according to the reference (including references cited therein) and by publicly known techniques.

eNOS activator

The eNOS activator used for the present invention is not particularly limited as long as it is an agent which improves the activity of nitric oxide synthase (NOS) or its substrate, and various eNOS activators can be used. Among them, preferred is L-arginine. NOS is an enzyme which produces NO from L-arginine, and endothelial NOS (eNOS) is constantly expressed in the vascular endothelium. Therefore, increasing the blood concentration of L-arginine, which is a substrate of eNOS, increases NO production and also the intracellular cGMP concentration in vascular endothelial cells.

cGMP or cGMP analog

The cGMP analog used for the present invention is a substance which has chemical modification in its chemical structure of cGMP (guanosine 3',5'-cyclic monophosphate) and which activates cGMP-dependent protein kinase. Various analogs described in publicly known references (for example, Corbin J. D., et al., J. Biol. Chem., 261 (3), 1208-1214, 1986) can be used, and preferred is 8-bromo cGMP (8-bromoguanosine 3',5'-cyclic monophosphate).

In the present invention, a "medicinal agent" means one which comprises a specified active ingredient and is used for the expression of a prescribed pharmacological effect. The form, composition, administration method, etc. are not limited. In the embodiments of the medicinal agent of the present invention, it is allowable that the active ingredient (s) is/are in a single product and that two or more products are used in combination to achieve the present invention.

In the present invention an "active ingredient" is one of the ingredients comprised in the medicinal agent, and such an active ingredient has at least a part of the desired pharmacological effect of the medicinal agent. The amount/content of the active ingredient in the medicinal agent is not particularly limited, and may be blended at various ratios depending on the strength of the pharmacological effect of the ingredient.

The substance used as an active ingredient in the medicinal agent of the present invention may be a pharmaceutically acceptable salt of the above-described substance having an activity of enhancing vascular endothelial intracellular cGMP, such as a GC-A agonist, and preferably a pharmaceutically acceptable salt of hANP, hBNP, hCNP-22, hCNP-53, hCNP6-22, or sildenafil. That is, in the present invention, the acid addition salts of the above mentioned substances may be used as an active ingredient. Examples of the acid include inorganic acids, such as hydrochloric acid, sulfuric acid, and phosphoric acid, and organic acids, such as formic acid, acetic acid, butanoic acid, succinic acid, and citric acid. Alternatively, in the present invention, the forms of metal salts, such as sodium salt, potassium salt, lithium salt, and calcium salt, and organic base salts of the above mentioned substances may be used as an active ingredient. The medicinal composition of the present invention may have the active ingredient in a free state and the active ingredient may be a pharmaceutically acceptable salt thereof. The most preferable sildenafil salt is the citrate.

The substance used as an active ingredient in the medicinal agent etc. of the present invention or a pharmaceutically acceptable salt thereof is preferably mixed with a publicly known pharmaceutically acceptable carrier, excipient, diluent, etc. to be formed into a medicinal composition, and administered to an individual by an administration method conventionally used in the pharmaceutical field, i.e., oral administration or parenteral administration, such as permucosal administration, intravenous administration, intramuscular administration, and subcutaneous administration.

In cases where the active ingredient is a peptide substance, it may be orally administered as a formulation resistant to degradation in the digestive tract, for example, a microcapsule formulation of liposomes encapsulating the peptide as the active ingredient. The administration can be performed not through the mucosa of the digestive tract but through, for example, the rectal, intranasal, sublingual mucosa. In this case, the active ingredient can be administered in the form of a suppository, a nasal spray, an inhalant, a sublingual tablet, etc. In the present invention, such formulations may be used that the peptide retention in the blood is improved by adopting various controlled-release formulations or long-acting formulations which comprise a biodegradable polymer represented by polysaccharide such as dextran, polyamine, PEG, etc. as the carrier.

The dosage amount of the substance used as an active ingredient of the medicinal agent of the present invention varies with the type of disease; the age, body weight, and degree of the symptoms of the individual (patient) ; and the route of administration, but generally the upper limit of the daily dosage of each active ingredient is, for example, about 100 mg/kg or less, preferably about 50 mg/kg or less, and more preferably 1 mg/kg or less. The lower limit of the daily dosage of each active ingredient is, for example, about 0.1 $\mu$g/kg or more, preferably 0.5 $\mu$g/kg or more, and more preferably 1 $\mu$g/kg or more.

The administration frequency of the medicinal agent (medicinal composition) of the present invention varies with the active ingredient to be used, the route of administration, and the specific disease to be treated. For example, in cases where a natriuretic peptide is orally administered, 4 times or less daily is preferred. In cases of parenteral administration, for example intravenous administration, continuous administration with the use of an infusion pump, a catheter, etc. is preferred.

In cases where the peptide, such as ANP or BNP, or a salt thereof is administered, for example, a lyophilized formulation thereof may be dissolved in water for injection, and then continuously administered with the use of a microinfusion pump (if not available, microinfusion set for children) . The duration of the continuous administration is several hours to several days (for example, about 3 to 14 days (preferably about 3 to 7 days). In this case, the upper limit of the dosage of each active ingredient may be appropriately selected in a concentration range of, for example, about 50 $\mu$g/kg/min (about 72 mg/kg/day) or less. The dosage may be about 5 $\mu$g/kg/min (about 7.2 mg/kg/day) or less, preferably about 0.5 $\mu$g/kg/min (about 720 $\mu$g/kg/day) or less, more preferably about 0.2 $\mu$g/kg/min or less, further more preferably about 0.1 $\mu$g/kg/min or less, and further more preferably about 0.05 $\mu$g/kg/min or less. The lower limit is about 0.0001 $\mu$g/kg·min (about 0.144 $\mu$g/kg/day) or more, and preferably about 0.001 $\mu$g/kg/min (about 1.44 $\mu$g/kg/day) or more. Specific administration method may be, for example, about 0.025 $\mu$g/kg/min for about 3 days or more (preferably 3 or 4 days), and in this case, the daily dosage in terms of the active ingredient is about 36 μg/kg.

Since a natriuretic peptide has an effect of relaxing and dilating blood vessels and decreasing the blood pressure as described above, in the prevention and/or therapy of the metastasis of malignant tumor cells, the administration is preferably performed at a rate not decreasing the blood pressure beyond necessity, and monitoring the blood pressure during and immediately after the administration is preferred. In this case, the duration of administration of hANP is usually 1 day or more. For this duration, the administration is preferably continued, and the duration is usually 1 day or more, preferably several days, and more preferably 1 to 5 days. In cases where malignant tumors should be controlled for a long period of time, the above-mentioned administration method may be appropriately repeated and the dosage, duration, etc. may be appropriately changed depending on the patient's conditions.

Regarding the dosage of the vascular endothelial intracellular cGMP enhancer of the present invention, it is preferred that the dosage and the administration method are selected so as to increase the intracellular cGMP concentration in the endothelial cells of peripheral blood vessels of the subject which received the administration, or increase the cGMP concentration in the peripheral blood. Such a dosage and an administration method can be appropriately selected by collecting the peripheral blood of the patient and monitoring the cGMP concentration.

Specifically, in intravenous administration of ANP, for example, it is preferred that 1000 μg of ANP (for example, HAMP for INJECTION 1000 manufactured by DAIICHI SANKYO Co., Ltd.) is dissolved in 10 mL of water for injection and then administered at a rate of "body weight × 0.06 mL/hour" (0.1 μg/kg/min or less). The administration rate is not limited to the above rate. Preferably, the administration rate is appropriately adjusted in the range of 0.2 μg/kg/min or less (preferably about 0.01 μg/kg/min or more) depending on the disease condition, with monitoring of the blood pressure and the heart rate. In particular, 3- or 4-day continuous administration of hANP at 0.025 μg/kg/min has been confirmed not to widely change physical conditions, such as the blood pressure and the heart rate, and thus this dosage and administration method is preferably employed.

In intravenous administration of BNP, for example, it is preferred that hBNP is continuously administered at about 0.1 μg/kg/min, and before the beginning of the administration, bolus administration of about 2 μg/kg of hBNP may be performed in combination. In this case also, the administration is preferably performed at a rate not decreasing the blood pressure beyond necessity, and monitoring the blood pressure during and immediately after the administration is recommended. In cases where the administration of ANP or BNP at the above rate does not have a significant impact on the blood pressure, heart rate, etc., the administration rate may be further increased. Regarding other active fragments, mutants, derivatives, mutants, etc. of ANP or BNP, the administration rate may be appropriately determined depending on the activity and the retention.

In cases where an active fragment, a mutant, a derivative, or a modified form of ANP, BNP, CNP, etc. is used instead of the naturally occurring type of peptide (hANP, hBNP, hCNP-22, etc.), the dosage, administration method, administration rate, administration frequency, etc. may be appropriately determined depending on the properties of the substance, such as strength of the activity, retention in the body, and molecular weight. Regarding the active ingredient, such as hANP, hBNP, and CNP (hCNP-22, hCNP-53), the use of controlled-release formulation technique, long-acting formulation technique, or various mutants, derivatives, modified forms, etc. resistant to peptide degradation allows employment of an administration method, administration frequency, etc. which inflicts less suffering on the patient, without limitation to bolus or continuous administration.

The timing of the administration may be any time after the detection of cancer. In view of general metastasis suppressing, continuous administration or regular administration at a certain intervals is preferred. In cases where surgery for cancer removal is performed, cancer recurrence can be significantly suppressed by the administration starting during the surgery and continued for several days.

An objective of the present invention is to suppress the metastasis of a malignant tumor, and the intended patients are usually those with a malignant tumor. The intended patients may be cancer patients without any other underlying diseases, or malignant tumor patients at high risk of heart failure, for example. The type of the malignant tumor is not particularly limited, and patients with any type of malignant tumor may receive the administration. Examples of the malignant tumor include various types, for example, epithelial malignant tumor, such as carcinoma; non-epithelial malignant tumor, such as sarcoma; and melanoma. The vascular endothelial intracellular cGMP enhancer of the present invention acts on vascular endothelium to suppress tumor cells from adhering to and infiltrating into vascular endothelium of a blood vessel or a lymphatic vessel (such adhesion and invasion is a common metastasis process to all tumor cells), and therefore is considered to exert an effect (indirect effect) of suppressing the metastasis of all malignant tumors regardless of the type of tumor. Therefore, even when tumor cells which do not express GC-A have become malignant and invade into a blood vessel or a lymphatic vessel, this indirect effects prevents the tumor cells from adhering to vascular endothelial cells or infiltrating into tissue, and thus inhibits the metastasis. Such an effect is supported by the fact that, in a tail vein injection metastasis mouse model, the administration of a GC-A agonist, a GC-B agonist, or a PDE5 inhibitor significantly suppressed the metastasis of tumor cells to each organ not only in the cases of A549 and H460 lung cancer cell lines expressing GC-A but also in the case of melanoma cells not expressing GC-A and having a high metastatic ability. Further, in a similar melanoma tail vein injection metastasis test using endothelial tissue-specific GC-A knockout mice, significantly increased metastasis as compared to that in wild type mice was confirmed. In addition, in the test using endothelial tissue-specific GC-A overexpression mice, significantly suppressed metastasis as compared to the case of wild type mice was confirmed. In GC-A knockout mice, the metastasis of melanoma was observed even in the heart, to which tumors do not usually metastasize. Therefore, it seems that among signals which enhance intracellular cGMP, in particular the signal mediated by GC-A prevents tumor cells from adhering to and infiltrating into vascular endothelial tissue.

The tumor may be a primary tumor or a metastatic tumor. In the case of a primary tumor, the administration is preferably performed in combination with a cancer removal surgery. Similarly, in the case of a metastatic tumor, if removal surgery is possible, the administration is preferably performed during surgery and following several days in combination with the surgery. If a resection surgery is impossible or metastasis to another organ is suspected, it is preferred to control metastasis by regular administration.

Further, in cases where a natriuretic peptide receptor GC-A is expressed in tumor cells of a malignant tumor patient, for example, in the case of an epithelial malignant tumor, it can be expected that the administration of a GC-A agonist exerts a further favorable metastasis-suppressing effect because the agonist can multidirectionally suppress the metastasis with both the direct effects including EMT suppression and indirect effects including prevention of adhesion to and invasion into vascular endothelium of a blood vessel. Examples of the malignant tumor include lung cancer, pancreatic cancer, thyroid cancer, breast cancer, uterine cancer, ovarian cancer, prostatic cancer, bone tumor, brain tumor, etc. To select a subject to receive the administration of the medicinal agent of the present invention, which can prevent tumor cells themselves from acquiring metastatic ability, harvest of tumor tissue may be performed before the administration to check whether GC-A is expressed in the cells. In cases where the expression of the receptor is confirmed, the agent may be administered to the patient.

The medicinal agent etc. further includes a method for preventing or suppressing the metastasis of a malignant tumor characterized in that two or more kinds of the vascular endothelial intracellular cGMP enhancers as active ingredients are used in combination, an administration method for the method, and/or a medicinal agent used for the method. A "medicinal agent for combined administration" of multiple active ingredients or drugs means that the active ingredients or drugs are intended to be administered in combination when the medicinal agent is used.

In the present invention, "combined administration" of multiple active ingredients or drugs means that a subject to receive the administration takes all the combined active ingredients or drugs into the body in a certain period of time. The active ingredients may be administered as a single formulation containing all the ingredients (so-called compounding agent). Alternatively, the active ingredients may be separately formulated into separate formulations and then separately administered (so-called combined administration). In cases where the active ingredients are separately formulated, the timing of the administration is not particularly limited. The formulations may be administered simultaneously, at certain time intervals, or on different days. In cases where two or more active ingredients are administered at different timings or on different days, the order of the administration of each active ingredient is not particularly limited. Normally, each formulation is administered according to each administration method, and therefore the frequency of the administration may be the same or different among the formulations . In cases where each active ingredient is separately formulated, the administration method (route of administration) may be the same or different among the formulations. It is not necessary that all the active ingredients are present in the body at the same time. As long as all the active ingredients are taken into the body during a certain period of time (for example, one month, preferably one week, more preferably several days, still more preferably one day), it is allowable that one active ingredient has already disappeared from the body when another active ingredient is administered.

In administering two or more kinds of the vascular endothelial intracellular cGMP enhancers in combination as active ingredients in the medicinal agent etc. of the present invention, when at least one kind of GC-A agonist is comprised as an active ingredient, at least one kind selected from the group consisting of a GC-B agonist, a NEP inhibitor, a PDE5 inhibitor, a NO donor, an eNOS activator, a GC-C agonist, and a cGMP analog may be combined. Preferred are combinations with at least one selected from a GC-B agonist, a NEP inhibitor, a PDE5 inhibitor, and a NO donor, and more preferred is a combination with a GC-B agonist or a PDE5 inhibitor. When at least one kind of GC-B agonist is used as an active ingredient, at least one kind selected from the group consisting of a NEP inhibitor, a PDE5 inhibitor, a NO donor, an eNOS activator, a GC-C agonist, and a cGMP analog may be combined. Preferred are combinations with at least one selected from a NEP inhibitor, a PDE5 inhibitor, and a NO donor, and more preferred is a combination with a NEP inhibitor or a PDE5 inhibitor. When at least one kind of PDE5 inhibitor is used as an active ingredient, at least one kind selected from the group consisting of a NEP inhibitor, a NO donor, an eNOS activator, a GC-C agonist, and a cGMP analog may be combined. Preferred is a combination with at least one kind selected from a NEP inhibitor and a NO donor.

In addition, when used in combination with at least one kind of conventionally used antitumor medicine, the medicinal agent of the present invention can efficiently treat a malignant tumor. Thus the present invention also provides a combined therapy with such an antitumor medicine. Since the medicinal agent of the present invention can control the metastasis or invasion of tumor cells, it is expected that appropriately administering the medicinal agent in combination during a therapy using another antitumor medicine can increase the efficiency of the therapy and improve the prognosis of the therapy. Examples of the antitumor medicine used in combination include an alkylating agent, an antimetabolite, an antitumor antibiotic, an antitumor plant constituent, a BRM (biological response control substance), a hormone, a vitamin, an anticancer antibody, a molecular target agent, and other antitumor medicines.

More specifically, examples of the alkylating agent include alkylating agents, such as nitrogen mustard, nitrogen mustard N-oxide and chlorambucil; aziridine alkylating agents, such as carboquone and thiotepa; epoxide alkylating agents, such as dibromomannitol and dibromodulcitol; nitrosourea alkylating agents, such as carmustine, lomustine, semustine, nimstine hydrochloride, streptozocin, chlorozotocin, and ranimustine; busulfan, improsulfan tosilate, dacarbazine, etc.

Examples of various antimetabolites include purine antimetabolites, such as 6-mercaptopurine, 6-thioguanine, and thioinosin; pyrimidine antimetabolites, such as fluorouracil, tegafur, tegafur-uracil, carmofur, doxifluridine, broxuridine, cytarabine, and enocitabine; folate antimetabolites, such as methotrexate and trimetrexate; etc.

Examples of the anticancer antibiotic include anthracycline antibiotic antitumor agents, such as mitomycin-C, bleomycin, peplomycin, daunorubicin, aclarubicin, doxorubicin, pirarubicin, THP-adriamycin, 4'-epidoxorubicin, and epirubicin; chromomycin A3, actinomycin-D; etc.

Examples of the antitumor plant constituent include vinca alkaloids, such as vindesine, vincristine, and vinblastine; taxanes, such as paclitaxel and docetaxel; epipodophyllotoxins, such as etoposide and teniposide; etc.

Examples of the BRM include a tumor necrosis factor, indomethacin, etc.

Examples of the hormone include hydrocortisone, dexamethasone, methylprednisolone, prednisolone, prasterone, betamethasone, triamcinolone, oxymetholone, nandrolone, methenolone, fosfestrol, ethinylestradiol, chlormadinone, medroxyprogesterone, etc.

Examples of the vitamin include vitamin C, vitamin A, etc.

Examples of the anticancer antibody and the molecular target agent include trastuzumab, rituximab, cetuximab, nimotuzumab, denosumab, bevacizumab, infliximab, imatinib mesilate, gefitinib, erlotinib, sunitinib, lapatinib, sorafenib, etc.

Examples of the other antitumor agents include cisplatin, carboplatin, oxaliplatin, tamoxifen, camptothecin, ifosfamide, cyclophosphamide, melphalan, L-asparaginase, aceglatone, sizofiran, picibanil, procarbazine, pipobroman, neocarzinostatin, hydroxyurea, ubenimex, krestin, etc.

In the present invention, when additional antitumor agents are administered in combination with the vascular endothelial intracellular cGMP enhancer, one or more kinds of the vascular endothelial intracellular cGMP enhancers and the additional antitumor agents may be contained in a single formulation or in different formulations. The order etc. of administration of one or more kinds of the vascular endothelial intracellular cGMP enhancers and the additional antitumor agents is not particularly limited.

The medicinal agent of the present invention can be provided as a kit. Examples of the kit for suppressing or preventing the metastasis of a malignant tumor, the kit comprising at least one kind of the vascular endothelial intracellular cGMP enhancer, include a kit comprising a vial in which a natriuretic peptide, such as ANP, BNP, or CNP, or a salt thereof is encapsulated as a lyophilized formulation and water for injection to be used for dissolving the formulation. In addition, a syringe to be used for dissolving and/or administering the formulation may be combined, and also a microinfusion pump or a microinfusion set for children may be combined. In cases where two or more kinds of the vascular endothelial intracellular cGMP enhancers as active ingredients are used in combination, vials, tablets, etc. each containing the active ingredient may be combined to form a kit.

In cases where the vascular endothelial intracellular cGMP enhancer of the present invention is a peptide substance such as a natriuretic peptide, gene therapy where the peptide is expressed in the patient's body as a result of introduction of a gene which encodes the peptide.

As the ANP gene, a gene comprising a nucleotide sequence which encodes the amino acid sequence of SEQ ID NO: 1 or 2 (for example, the one described in Science, vol. 226, 1206, 1984) may be used. As the BNP gene, a gene comprising a nucleotide sequence which encodes the amino acid sequence of SEQ ID NO: 3, 4, or 5 (for example, the one described in Biochem. Biophys. Res. Commun., vol. 165, 650, 1989) may be used. As the CNP gene, a gene comprising a nucleotide sequence which encodes the amino acid sequence of any of SEQ ID NOs: 7 to 10 (for example, the one described in Biochem. Biophys. Res. Commun., vol. 165, 650, 1989) may be used. When the above-mentioned gene is used for therapy, the gene may be introduced via intramuscular injection or local injection, using a retrovirus, an adenovirus, an adeno-associated virus, or an artificial vector as a vector. Alternatively, without the use of such a vector, the gene may be introduced in the form of a plasmid. Specific gene therapy may be performed by the method described in Jikken Igaku (Experimental Medicine) vol. 12, 303, 1994 or a method described in the references cited therein, etc.

The present invention further includes a medicinal agent for suppressing or preventing the metastasis of a malignant tumor, the agent comprising at least one kind of vascular endothelial intracellular cGMP enhancer as an active ingredient to be administered to a subject receiving the administration of a different kind of vascular endothelial intracellular cGMP enhancer; and a method for suppressing or preventing the metastasis of a malignant tumor, the method comprising administering an effective amount of one kind of vascular endothelial intracellular cGMP enhancer to a subject receiving therapy by the administration of a different kind of vascular endothelial intracellular cGMP enhancer.

The vascular endothelial intracellular cGMP enhancers as active ingredients, the administration method, etc. are the same as those described for the above medicinal compositions. In such administration, a preferred combination of the vascular endothelial intracellular cGMP enhancers is the combination of a GC-A agonist or a GC-B agonist and a PDE5 inhibitor, more preferred is the combination of hANP, hCNP-22, hCNP6-22 or a derivative thereof and sildenafil or a pharmacologically acceptable salt thereof, and still more preferred is the combination of hANP and sildenafil citrate.

Examples

Hereinafter, the invention will be specifically described by referring to the Examples below. The Examples are merely illustrative examples of the embodiments of the present invention, and the present invention is not limited thereto.

The experimental materials used in the Examples below were obtained and prepared as follows.

hANP and hCNP were obtained from Asubio Pharma Co., Ltd. and hBNP was obtained from Peptide Institute, Inc. (Osaka), each in the form of a lyophilized product. Each of the lyophilized products was dissolved in physiological saline containing IBMX. After the concentration was adjusted as needed so that the final concentration of IBMX would be $5\times10^{-4}$ M, each solution was used in the following experiments.

Lung adenocarcinoma cell line, A549 cells, was obtained from ATCC (Manassas, VA). The cells were cultured in DMEM culture medium (Invitrogen, Carlsbad, VA) containing 10% FCS under 5% $CO_2$ at 37°C. The amounts of the culture medium were 1.5 mL for each well of a 6-well dish, 400 $\mu$L for each well of a 24-well dish, and 150 $\mu$L for each well of a 96-well dish. In the Examples below, unless otherwise stated, the same culture medium and culture conditions were adopted.

TGF-$\beta$1 (transforming growth factor $\beta$1) was purchased from R&D systems Inc. (Minneapolis, MN) and diluted with physiological saline as needed and used for the following experiments.

Example 1

Changes in the intracellular cGMP level in response to stimulus of natriuretic peptide on cancer cells

The A549 cells were cultured in a 24-well dish at $4\times10^4$ cells/well, and the culture medium was replaced with DMEM culture medium free from FCS the next day. To the culture medium, solutions of hANP, hBNP, and hCNP (final concentration: ten-fold serial dilution from $1\times10^{-11}$ M to $1\times10^{-6}$ M, the same amount of physiological saline was used as a control) were separately added. After 10 minutes passed, 400 $\mu$L of cooled 70% ethanol (containing 0.1 N hydrochloric acid) was added, and ultrasonication was performed for a short time. The treated solution was lyophilized, and the cGMP level was measured using Cyclic GMP Assay kit (made by Yamasa Corp.). The cGMP level measurement results are shown in Fig. 1.

hANP and hBNP, which are GC-A agonists, increased the intracellular cGMP level of the A549 cells in a concentration-dependent manner. Meanwhile, hCNP, which is a CG-B receptor agonist that does not bind to the GC-A receptor, did not show such increase. Thus, it was revealed that the signaling in A549 cells is attributable to the response specific to stimulus from the GC-A receptor.

Example 2

Growth-suppression effect of natriuretic peptides on A549 cells

Using a 24-well dish, the A549 cells were cultured at $4\times10^4$ cells/well, and the culture medium was replaced with DMEM culture medium free from FCS the next day. To the culture medium, solutions of hANP, hBNP, and hCNP (final concentration: ten-fold serial dilution from $1\times10^{-9}$ M to $1\times10^{-6}$ M, the same amount of physiological saline was used as a control) were separately added. After 24 and 48 hours passed, cell growth assay was performed with the use of Cell Count Reagent SF (made by Nacalai Tesque). The results are shown in Fig. 2.

Until 48 hours, the A549 cell counts of hANP, hBNP, and hCNP solutions were similar to those of the control even at the highest concentration of $1\times10^{-6}$ M. According to Vesely et al., ANP has an effect of suppressing the growth of various cancer cells derived from pancreatic cancer, breast cancer, etc. However, in the experimental system of the present invention, none of the natriuretic peptides at $1\times10^{-6}$ M (1 $\mu$M) had any effect on the growth of cancer cells.

Example 3

The effect of hANP on EMT induced by TGF-β1

Using a 6-well dish, the A549 cells were cultured at $1{\times}10^5$ cells/well, and the culture medium was replaced with DMEM culture medium free from FBS the next day. After 24 hours of culture, the cells were used in the following experiment.

An ANP group (ANP solution was added (final concentration: 1 μM)) and a control group (the same amount of physiological saline was added) were prepared, and after 2 hours from the addition, TGF-β1 was added so that the final concentration would be 0.125, 0.25, 0.5, 1.0, and 2.0 ng/mL. After 24 hours had passed, the morphological change in the cells was observed under a microscope and photographed (Fig. 3).

Then, total RNA was collected using TRIZOL total RNA isolation reagent (made by Invitrogen), cDNA was synthesized using reverse transcriptase, and the mRNA levels of E-cadherin, N-cadherin, VEGF-A, PDGF-B, TNF-α, and IL-6 were measured by quantitative RT-PCR. The results of the mRNA level measurement are shown in Fig. 4. It is known that in EMT, the expression of E-cadherin decreases while the expression of N-cadherin increases. The expression ratio of N-cadherin/E-cadherin is considered to be useful as an indicator of the degree of EMT. The ratios are shown in Fig. 5.

As shown in Fig. 3, 1 μM of hANP (Fig. 3C) significantly suppressed the morphological change (change to spindle shape similar to that of mesenchymal components as shown in Fig. 3B) associated with EMT induced by the TGF-β1 stimulus. Also, as shown in Fig. 4, hANP significantly suppressed the gene expression of growth factors (VEGF-A, PDGF-B) and inflammatory cytokines (TNF-α, IL-6) increased by EMT induced by the TGF-β1 stimulus. Also, hANP significantly suppressed increase in the expression of N-cadherin induced by the TGF-β1 stimulus and inhibited decrease in the expression of E-cadherin induced by the same stimulus. As a result, as shown in Fig. 5, the expression ratio of N-cadherin/E-cadherin, which is an important indicator of EMT, significantly decreased in the presence of 1 μM hANP. The same experiment using H460 cells derived from large cell lung carcinoma showed similar results.

Thus, hANP at a concentration of 1 μM showed the effect of significantly suppressing EMT, which is an important phenomenon triggering the metastasis of cancer. As shown in Example 2, hANP at 1 μM hardly has an influence on the growth of the cancer cells. Therefore, it seems that hANP suppresses the EMT of cancer cells by a completely different mechanism from that regarding the effect on cancer growth.

Example 4

Effect of hANP on migration ability (Boyden Chamber Assay) of A549 cells

Using a 6-well dish, the A549 cells were cultured at $1{\times}10^5$ cells/well, and the culture medium was replaced with DMEM culture medium free from FBS the next day. After 24 hours of culture, the cells were divided into 3 groups (the control group, the TGF-β1 group, and the TGF-β1 + hANP group), and used in the following experiment.

To the culture medium for the TGF-β + hANP group, the hANP solution was added so that the final concentration of hANP might be 1 μM. For the control group and the TGF-β1 group, the same amount of physiological saline was added to the culture medium. After 2 hours from the addition, the Boyden chamber assay was performed as follows.

DMEM culture medium free from FBS for the control group, the same DMEM culture medium containing TGF-β1 (final concentration: 1 ng/mL) for the TGF-β1 group, and the same DMEM culture medium containing hANP (final concentration: 1 μM) and TGF-β1 (final concentration: 1 ng/mL) for the TGF-β1 + hANP group were separately added to each well of a 6-well dish in an amount of 1.5 mL. On the surface of each liquid, a cell culture insert (8.0 μm pore size, made by Becton, Dickinson and Company) was placed, and onto each cell culture insert, 1.5 mL of a cell suspension of A549 cells of each group in FBS-free DMEM (hANP at final concentration of 1 μM was added for the TGF-β1 + hANP group) was added and cultured. Twenty hours later, the cell culture insert of each group was fixed with 10% formalin, the upper surface was wiped, and only the lower surface was stained with hematoxylin and eosin (H&E). The number of A549 cells which adhered to the lower surface of the insert was observed under a microscope. Fig. 6A shows the photographs of the cells, and Fig. 6B shows a graph of the number of cells. The migration ability of A549 cells significantly increased in the TGF-β1 group, but this phenomenon was significantly suppressed by 1 μM hANP. The same experiment using H460 cells derived from large cell carcinoma showed similar results.

Example 5

Effect of hANP on motility (Wound Healing Assay) of cancer cells

Using a 6-well dish, the A549 cells were cultured at $4{\times}10^5$ cells/well, and the culture medium was replaced with DMEM culture medium free from FBS the next day. After 24 hours of culture, the cells were divided into 3 groups (the control group, the TGF-β1 group, and the TGF-β1 + hANP group), and used in the following experiment.

To the culture medium for the TGF-β1 + hANP group, the hANP solution was added so that the final concentration of hANP would be 1 μM. For the control group and the TGF-β1 group, the same amount of physiological saline was added to the culture medium. Two hours later, a wound was created at the center of each well with the use of a 200 μL-pipette tip. Immediately after the wound creation, a photograph of each well was taken under a microscope (Fig. 7A). To the TGF-β + hANP group and the TGF-β1 group, TGF-β1 was added so that the final concentration would be 1 ng/mL. After 24-hour culture, a photograph of the same part was taken again to calculate the average ratio of the wounded area width difference between immediately after the wound creation and after 24-hour culture (migration ratio: % wounded area filled) by the following formula.

```
Migration ratio (%) = (initial wound width - wound width after

    24-hour culture) / (initial wound width) × 100
```

Fig. 7 shows the photograph of the wounded area of each well after 24 hours, and Fig. 8 shows the migration ratio of each group.

The motility of A549 cells was significantly increased by the TGF-β1 stimulus, but this increase was significantly suppressed in the presence of 1 μM hANP. The same experiment using H460 cells derived from large cell lung carcinoma showed similar results.

Example 6

The action of hANP on invasion ability of cancer cells

Using a 6-well dish, the A549 cells were cultured at $1 \times 10^5$ cells/well, and the culture medium was replaced with DMEM culture medium free from FBS the next day. After 24 hours of culture, the cells were divided into 3 groups (the control group, the TGF-β1 group, and the TGF-β1 + hANP group), and used in the following experiment.

To the culture medium for the TGF-β1 + hANP group, the hANP solution was added so that the final concentration of hANP would be 1 μM. For the control group and the TGF-β1 group, the same amount of physiological saline was added to the culture medium. After 2 hours from the addition, the Boyden chamber assay was performed as follows. Cell culture inserts (8.0 μm pore size, made by Becton, Dickinson and Company) coated with Matrigel were prepared beforehand by placing cell culture inserts on a 6-well dish, adding 300 μL each of a 0.5 μg/μL Matrigel solution (adjusted with PBS) thereonto, and air drying the inserts.

DMEM culture medium free from FBS for the control group, the same DMEM culture medium containing TGF-β1 (final concentration: 1 ng/mL) for the TGF-β1 group, and the same DMEM culture medium containing hANP (final concentration: 1 μM) and TGF-β1 (final concentration: 1 ng/mL) for the TGF-β1 + hANP group were separately added to each well of a 6-well dish in an amount of 1.5 mL. On the surface of each liquid, the cell culture insert coated as above was placed, and onto each cell culture insert, 1.5 mL of a cell suspension of A549 cells of each group in FBS-free DMEM (hANP at final concentration of 1 μM was added for the TGF-β1 + hANP group) was added and cultured.

After 40-hour culture, the cell culture insert of each group was fixed with 10% formalin, the upper surface was wiped, and only the lower surface was stained with hematoxylin and eosin (H&E). The number of A549 cells which adhered to the lower surface of the insert was observed under a microscope. Fig. 9A shows the photographs of the cells, and Fig. 9B shows a graph of the number of the cells.

The cell invasion via the Matrigel layer significantly increased in the TGF-β1 group, but this cell invasion was significantly suppressed by 1 μM hANP. The same experiment using H460 cells derived from large cell lung carcinoma showed similar results.

Example 7

The apoptosis-inducing activity of hANP in cancer cells

Using a 96-well dish, the A549 cells were cultured at $1 \times 10^4$ cells/well. The culture medium was replaced with DMEM culture medium free from FBS the next day, and after 24-hours culture, the following experiment was conducted.

To the culture medium, a hANP solution prepared by serial dilution was added so that the final concentration of hANP would be 1, 5, or 10 μM (for the control group, the same amount of physiological saline was added). Further, 2, 4, and 6 hours later, the same amounts of the hANP solutions at the same concentrations were added (for the control group, the same amount of physiological saline was added) (4 times in total) . 48 hours later, trypan blue staining was performed and the cell viability (number of living cells/total number of all cells) was determined with the use of an automatic cell counter Countess (registered trademark) (Invitrogen). The same experiments using H460 cells derived from large cell lung carcinoma, H520 cells derived from squamous cell lung carcinoma, and H358 cells derived from bronchoalveolar carcinoma showed similar results. The results are shown in Fig. 10.

The number of the living cancer cells of various kinds decreased in a hANP-concentration-dependent manner, showing that continuous presence of hANP in the culture medium induces apoptosis in cancer cells.

Example 8

Combined effects of hANP and cisplatin on apoptosis induction in cancer cells

As the cisplatin, CISPLATIN inj. "Maruko" (trade name, Nichi-Iko Pharmaceutical) was used.

Using a 96-well dish, the A549 cells were cultured at $1 \times 10^4$ cells/well. The next day, the culture medium was replaced with DMEM culture medium free from FBS. After 24-hours culture, the cells were divided into groups (control group, cisplatin single administration group, 1 $\mu$M hANP group, cisplatin + 1 $\mu$M hANP group, 10 $\mu$M hANP group, and cisplatin + 10 $\mu$M hANP group), and the following experiment was conducted.

To the culture medium, for the hANP addition groups, a hANP solution prepared so that the final concentration of hANP would be 1 or 10 $\mu$M was added, whereas for the control group and the cisplatin single administration group, the same amount of physiological saline was added. Further, for the cisplatin addition groups, a cisplatin solution prepared so that the final concentration of cisplatin would be 1, 5, 10, 20, or 30 $\mu$M was added (for the control group and the hANP single administration group, the same amount of physiological saline was added). Further, 2, 4, and 6 hours later, the same amounts of the hANP solutions at the same concentrations were added (4 times in total of hANP addition) (for the control group and the cisplatin single administration group, the same amounts of physiological saline was added). 48 hours later, trypan blue staining was performed and the cell viability (number of living cells/total number of all cells) was determined with the use of an automatic cell counter Countess (registered trademark) (Invitrogen) . The same experiments were conducted using H460 cells, H520 cells, and H358 cells. The results for 1 $\mu$M hANP are shown in Fig. 11, and the results for 10 $\mu$M hANP are shown in Fig. 12.

It was revealed that the apoptosis-inducing activity of hANP in cancer cells is enhanced when used in combination with cisplatin.

Example 9

Test for implantation of cancer cells into vascular endothelial cell system

Normal human umbilical cord vein endothelial cells (HUVEC) were purchased from Takara Bio, Inc. Medium preparation and subculture were performed according to the package insert. Nuclear staining was performed using TOPRO3 (purchased from Invitrogen), and immunostaining of VE-cadherin was performed using anti-VE-cadherin antibody (purchased from BD Biosciences).

After culturing the HUVEC to confluent and replacing the medium with a serum free medium, nuclear staining and immunostaining using anti-VE-cadherin antibody were performed in the presence or absence of VEGF and hANP to observe the expression of VE-cadherin. Fig. 13 shows photographs of stained cells under different conditions.

In the presence of VEGF alone, the expression of VE-cadherin decreased, and intercellular gaps were observed. However, in the presence of VEGF + hANP, this phenomenon was suppressed, and intercellular adhesion was well retained to the same degree as in the control group. Further, in the presence of VEGF alone, when A549 cells were added onto the HUVEC cell layer, implantation of the A549 cells into the HUVEC cell layer was observed. However, in the presence of VEGF + hANP (1 $\mu$M), this phenomenon of implantation was suppressed.

From the facts, it is assumed that hANP has the action of suppressing cancer cell implantation into vascular endothelial tissue and cancer cell invasion into tissue prone to metastasis .

Comprehensively, hANP is considered to have both direct effects and indirect effects on cancer cells, the direct effects including EMT suppression leading to decrease in the infiltrative or metastatic ability of cancer cells, and induction of cancer cell-specific apoptosis, the indirect effects including implantation-suppressing effect on vascular endothelial cells to be metastasized.

Example 10

Cancer recurrence-suppressing effect of hANP after cancer resection surgery in lung cancer patients

It is reported that lung cancer patients whose BNP level in the blood is 30 pg/mL or higher are at high risk of postsurgical complications after a cancer removal surgery. In this experiment, lung cancer patients were measured for the BNP level in the blood before cancer removal surgery. A part of the patients having the BNP level in the blood of 30 pg/mL or higher and patients judged to be at high risk of postsurgical complications by the doctor in charge were assigned to the hANP group (52 patients in total; cancer stage breakdown was IA: 26 patients (50%), IB: 16 patients (23%), II: 7 patients (13%), and III: 3 patients (6%)). The control group consisted of patients assigned regardless of the BNP level in the blood (287 patients in total, cancer stage breakdown was IA: 155 patients (54%), IB: 65 patients (23%), II: 35 patients (12%), and III: 32 patients (11%)) . To the hANP group, hANP (trade name: HAMP for INJECTION 1000, non-bolus, made by DAIICHI SANKYO Co., Ltd.) was continuously administered at 0.025 $\mu$g/kg/min starting during the surgery of lung cancer and the administration continued for 3 days after surgery. Before the surgery and for 4 days after surgery, each patient was measured for the blood pressure (systolic and diastolic), the heart rate, and the urine volume. (The results are shown in Fig. 15.)

For about 2 years after surgery, follow-up survey was conducted in regard to early recurrence of cancer in the patients. The patients annually underwent chest CT, head MRI, bone scintigraphy, or positron tomography (PET), and whole body checkup for recurrence. Fig. 14 shows the cancer recurrence-free survival rate of each group.

The cancer recurrence-free survival rate of the control group in 2 years after surgery decreased in accordance with the cancer stage before the surgery (IA: 91.6%, IB: 73.4%, II: 48%, III: 29%). In contrast, in the hANP group, early recurrence was not observed regardless of the cancer stage before the surgery. Also, as shown in Fig. 15, a low dosage therapy using hANP at 0.025 ($\mu$g/kg/min did not widely change physical conditions, such as the blood pressure, the heart rate, and the urine volume, etc. of the patients. The above facts suggested it is suggested that this dosage does not cause the living body any problematic changes resulting from the primary medicinal effect of hANP, even when administered to a patient without cardiovascular abnormality.

Therefore it is indicated that the low dosage hANP therapy is extremely useful as a safe anti-metastasis therapy with fewer side effects for not only cancer patients at a risk of heart failure but also cancer patients without a risk of heart failure.

Example 11

The actions of various GC-A agonists on EMT of A549 cells

Experiments were conducted in the same manner as in Example 3 except that instead of hANP as a test substance, hBNP (final concentration: 1 $\mu$M), CD-NP (final concentration: 10 $\mu$M (see Deborah et al., J. Biol. Chem., vol. 289, No. 50, 35003 -35009, 2008), and LKL-ANP (final concentration: 1 $\mu$M (a mutant in which the amino acids at positions 10 to 12 of hANP are replaced with LKL (see Furuya, M. et al., Biochem. Biophys. Res. Commun., 183 (3), 964-969, 1992) were used. As a result, in terms of the value of E-cad/Ncad, the ratio of suppression of EMT induced by TGF-$\beta$ was 44.8% (BNP), 37.7% (CD-NP), and 52.9% LKL-ANP). Thus, various kinds of the GC-A agonists showed an action of suppressing the EMT of tumor cells.

Example 12

The effect of hANP on metastasis of A549 cells in a mouse tail vein injection metastasis test

Six-week-old male Balb/c (nu/nu) mice were used (purchased from Japan SLC, Inc.).

The osmotic pumps used were MODEL2004 (for 28-day administration) made by ALZET (Cupertino, CA).

Mice into which an osmotic pump containing 0.9% physiological saline was subcutaneously implanted in the back were assigned to the control group. Similarly, mice into which an osmotic pump containing hANP prepared for administration at 0 .5 $\mu$g/kg/min was subcutaneously implanted were assigned to the ANP group. Injection of a tumor cell suspension was performed on the day after the osmotic pump implantation.

As the tumor cells, A549 cell line labeled with GFP (Green Fluorescent Protein) was purchased from Wako Pure Chemical Industries, Ltd., and cultured in RPMI1640 (Life Technologies Corp.) containing 10% FCS under 5% $CO_2$ at 37°C. The cells in a subconfluent state were treated with EDTA-trypsin, centrifuged, and then suspended in serum free DMEM so as to be $1 \times 10^7$ cells/mL. The A549 cell suspension (100 $\mu$L/body, $1 \times 10^6$ cells) was injected to the tail vein of the mice. The administration of physiological saline or ANP with the use of the osmotic pump was continued for 4 weeks, and the condition of the lung metastasis 8 weeks after the tumor cell injection was observed with a fluorescence microscope (Olympus OV100) (Fig. 16). Fig. 17 shows the number of nodules formed due to lung metastasis per individual observed under a fluorescence microscope.

As shown in Figs. 16 and 17, the metastasis of cancer cells was significantly suppressed by the administration of hANP for the first 4 weeks.

The same experiment using H460 cells as the tumor cells showed similar results.

Example 13

The actions of various cGMP enhancers on the metastasis of melanoma in a mouse tail vein injection metastasis test

6-week-old male C56BL/6N mice were used (purchased from Japan SLC, Inc.). Mouse melanoma B16-F10 was purchased from ATCC, and cultured in DMEM (Life Technologies Corp.) containing 10% FCS under 5% $CO_2$ at 37°C. The cells in a subconfluent state were treated with EDTA-trypsin, centrifuged, and then suspended in serum free DMEM so as to be $5 \times 10^6$ cells/mL. The melanoma cell suspension (100 μL/mouse, $5 \times 10^5$ cells) was injected to the tail vein of the mice. The osmotic pumps used were MODEL2002 (for 14-day administration) made by ALZET (Cupertino, CA).

Mice into which an osmotic pump containing 0.9% physiological saline was subcutaneously implanted in the back were assigned to the control group. Similarly, an osmotic pump containing hANP prepared for administration at 0.5 μg/kg/min was subcutaneously implanted into each mouse in the ANP group, an osmotic pump containing mouse BNP prepared for administration at 0.5 μg/kg/min was subcutaneously implanted into each mouse in the BNP group, and an osmotic pump containing hCNP-22 prepared for administration at 2.5 μg/kg/min was subcutaneously implanted into each mouse in the CNP group. To the mice in the sildenafil group, sildenafil citrate at a dosage of 20 mg/kg was intraperitoneally administered once daily. Injection of a tumor cell suspension was performed as above on the day after the start of the administration, and the administration was continued for 2 weeks. The condition of the lung metastasis of the tumor cells 2 weeks after the tumor cell injection was observed (Fig. 18). Fig. 19 shows the number of observed nodules formed due to lung metastasis per individual.

As shown in Figs. 18 and 19, in all of the ANP group, the BNP group, the CNP group, and the sildenafil group, the metastasis of melanoma was significantly suppressed as compared with the control group.

Example 14

Melanoma tail vein injection metastasis test using GC-A knockout mice

A melanoma lung metastasis test was conducted in the same manner as in Example 13 except that the mice used were 12-week-old male GC-A knockout (KO) mice (whole body KO, endothelial tissue-specific KO, Tokudome T., Kishimoto I., Kangawa K., et al., Arterioscler Thromb Vase Biol. 2009; 29: 1516-21; Kishimoto I, Tokudome T, Nakao K, and Kangawa K. FEBS J. 2011; 278: 1830-41) and that the melanoma B16-F10 cell suspension was prepared at $2.5 \times 10^6$ cells/mL and 100 μL/mouse was injected. Fig. 20 shows a graph showing the numbers of nodules formed by the lung metastasis of the melanoma cells at 2 weeks from the injection in the wild type mice, the whole body GC-A KO mice, and the endothelial tissue-specific GC-A KO mice.

As shown in Fig. 20, in both of the whole body GC-A KO mice and the endothelial tissue-specific GC-A KO mice, the lung metastasis of melanoma significantly increased as compared with the wild type mice.

Example 15

Melanoma tail vein injection metastasis test using endothelial cell-specific GC-A overexpression mice

A tail vein injection metastasis test was conducted in the same manner as in Example 13 except that the mice used were 12-week-old male endothelial tissue-specific GC-A overexpression mice (prepared in reference to Zhun ML, et al., Cardiovasc Res. 2009; 84: 292-9, etc.) and that the melanoma B16-F10 cell suspension was prepared at $1.0 \times 10^7$ cells/mL and 100 μL/mouse was injected. Fig. 21 shows a graph showing the numbers of nodules formed in the lung at 2 weeks from the injection in the wild type mice and the GC-A overexpression mice.

As shown in Fig. 21, in the endothelial tissue-specific GC-A overexpression mice, contrary to the KO mice, the lung metastasis of melanoma was significantly suppressed as compared with the wild type mice.

The results of Examples 14 and 15 strongly suggest that the signaling mediated by GC-A, in particular in endothelial tissue in the living body, suppresses the metastasis of tumors.

**Claims**

1. A natriuretic peptide receptor GC-B agonist for use in preventing or suppressing the metastasis of a malignant tumor, wherein the natriuretic peptide receptor GC-B agonist is any one selected from the following (b1) to (b5) or a pharmacologically acceptable salt thereof and has an agonist activity for the natriuretic peptide receptor GC-B:

(b1) a c-type natriuretic peptide consisting of the amino acid sequence of SEQ ID NO: 7, 8, 9 or 10,
(b2) a substance comprising an active fragment, wherein it has the amino acid sequence of SEQ ID NO: 11, or wherein it consists of the amino acid sequence from position 6 to position 22 of SEQ ID NO: 7,
(b3) a mutant wherein it is

(i) a peptide consisting of the amino acid sequence of SEQ ID NO: 7, 9 or 10 having substitution, deletion, insertion, and/or addition of one to several amino acids selected from positions 1 to 5,
(ii) a peptide consisting of the amino acid sequence of SEQ ID NO: 8 having substitution, deletion, insertion, and/or addition of one to several amino acids at any one to several positions selected from positions 1 to 36,

(b4) a derivative comprising any one of the amino acid sequences of SEQ ID Nos: 7 to 11,
wherein the derivative preferably further comprises at least one of the Fc region of an immunoglobulin, a serum albumin, and the C-terminus of ghrelin, and
(b5) a modified form wherein it comprises any one of the amino acid sequences of SEQ ID Nos: 7 to 10 of the sequence listing, and at least one amino acid other than the amino acids shown in SEQ ID NO: 11 is chemically modified, or wherein it is prepared by chemical modification by addition of a pharmaceutically usable polymer.

2. The natriuretic peptide receptor GC-B agonist for use of claim 1, wherein the natriuretic peptide receptor GC-B agonist is continuously used at 0.5 $\mu$g/kg/min or less for three to seven days.

3. The natriuretic peptide receptor GC-B agonist for use of claim 1, wherein the natriuretic peptide receptor GC-B agonist is continuously used at 0.1 $\mu$g/kg/min or less for one to several days.

4. The natriuretic peptide receptor GC-B agonist for use of claim 1, wherein the natriuretic peptide receptor GC-B agonist is used in suppressing or preventing the metastasis of a malignant tumor in a patient who undergoes surgery for removal of a tumor and, wherein the natriuretic peptide receptor GC-B agonist is continuously used from before the surgery to 1 to several days after surgery.

5. The natriuretic peptide receptor GC-B agonist for use of claim 1, wherein a peptide consisting of any of the amino acid sequences of SEQ ID NOs: 7 to 10 is continuously used at a dosage of 0.05 $\mu$g/kg/min or less from before the tumor-removing surgery for a certain period of time after surgery.

6. The natriuretic peptide receptor GC-B agonist for use of claim 4, wherein a peptide consisting of any one of the amino acid sequence of SEQ ID NOs: 7 to 10 is continuously used at a dosage of 0.025 $\mu$g/kg/min starting during the surgery and continued for 3 days after surgery.

7. The natriuretic peptide receptor GC-B agonist for use of claim 1, wherein two or more kinds of the natriuretic peptide receptor GC-B agonist as active ingredients are used in combination.

8. The natriuretic peptide receptor GC-B agonist for use of claim 1, wherein the implantation and/or invasion of tumor cells into vascular endothelial tissue is inhibited in a patient who received the natriuretic peptide receptor GC-B agonist.

9. The natriuretic peptide receptor GC-B agonist for use of claim 1, which is used in a subject at risk of developing an epithelial malignant tumor to provide the subject with at least one pharmacological effect selected from suppression of epithelial to mesenchymal transition (EMT) of tumor cells, inhibition of the acquisition of migration ability by tumor cells, suppression of the acquisition of motility by tumor cells, suppression of invasion by tumor cells, induction of tumor cell-specific apoptosis, and inhibition of implantation by tumor cells into vascular endothelial tissue.

10. The natriuretic peptide receptor GC-B agonist for use of claim 1, wherein the patient undergoes surgery for removal of a malignant tumor.

11. The natriuretic peptide receptor GC-B agonist for use of claim 10, wherein the patient receives administration of the natriuretic peptide receptor GC-B agonist for several days from the day before surgery.

**12.** The natriuretic peptide receptor GC-B agonist for use of claim 10, wherein the patient receives administration of the natriuretic peptide receptor GC-B agonist a day before the surgery.

**13.** The natriuretic peptide receptor GC-B agonist for use of claim 10, wherein the natriuretic peptide receptor GC-B agonist is administered to the patient by intramuscular administration or subcutaneous administration.

**14.** The natriuretic peptide receptor GC-B agonist for use of any one of claims 1 to 13, wherein the natriuretic peptide receptor GC-B agonist is used in combination with cisplatin.

**15.** The natriuretic peptide receptor GC-B agonist for use of claim 14, wherein the natriuretic peptide receptor GC-B agonist is an atrial natriuretic peptide consisting of any one of the amino acid sequence of SEQ ID NOs: 7 to 10.

**Patentansprüche**

**1.** Natriuretischer Peptidrezeptor-GC-B-Agonist zur Verwendung bei der Verhinderung oder Unterdrückung der Metastasenbildung eines bösartigen Tumors, wobei der natriuretische Peptidrezeptor-GC-B-Agonist aus einem der Folgenden (b1) bis (b5), oder einem pharmakologisch annehmbaren Salz davon ausgewählt ist und eine agonistische Aktivität für den natriuretischen Peptidrezeptor-GC-B hat:

(b1) einem C-Typ-natriuretischen Peptid, das aus der Aminosäuresequenz der SEQ ID Nr. 7, 8, 9 oder 10 besteht,
(b2) einer Substanz, die ein aktives Fragment umfasst, das die Aminosäuresequenz der SEQ ID Nr. 11 hat, oder das aus der Aminosäuresequenz von Position 6 bis Position 22 der SEQ ID Nr. 7 besteht,
(b3) einer Mutante, die

(i) ein Peptid ist, bestehend aus der Aminosäuresequenz der SEQ ID Nr. 7, 9 oder 10, das eine Substitution, Deletion, Insertion und/oder Addition von einer bis zu mehreren Aminosäuren ausgewählt aus den Positionen 1 bis 5 hat,
(ii) ein Peptid ist, bestehend aus der Aminosäuresequenz der SEQ ID Nr. 8, das eine Substitution, Deletion, Insertion und/oder Addition von einer bis zu mehreren Aminosäuren an einer bis zu mehreren Positionen der Positionen 1 bis 36 hat,

(b4) einem Derivat, das eine der Aminosäuresequenzen der SEQ ID Nrn. 7 bis 11 umfasst,
wobei das Derivat vorzugsweise weiterhin mindestens ein Element ausgewählt aus einer Fc-Region eines Immunglobulins, einem Serumalbumin und dem C-Terminus von Ghrelin umfasst, und
(b5) einer modifizierten Form, wobei diese eine der Aminosäuresequenzen der SEQ ID Nrn. 7 bis 10 des Sequenzprotokolls umfasst und in der mindestens eine andere Aminosäure als die in der SEQ ID Nr. 11 gezeigten Aminosäuren chemisch modifiziert ist, oder
wobei diese durch chemische Modifikation durch Zugabe eines pharmazeutisch verwendbaren Polymers hergestellt ist.

**2.** Natriuretischer Peptidrezeptor-GC-B-Agonist zur Verwendung nach Anspruch 1, wobei der natriuretische Peptidrezeptor-GC-B-Agonist kontinuierlich bei 0,5 μg/kg/min oder weniger für 3 bis 7 Tage verwendet wird.

**3.** Natriuretischer Peptidrezeptor-GC-B-Agonist zur Verwendung nach Anspruch 1, wobei der natriuretische Peptidrezeptor-GC-B-Agonist kontinuierlich bei 0,1 μg/kg/min oder weniger für einen bis zu mehreren Tagen verwendet wird.

**4.** Natriuretischer Peptidrezeptor-GC-B-Agonist zur Verwendung nach Anspruch 1, wobei der natriuretische Peptidrezeptor-GC-B-Agonist zur Unterdrückung oder Verhinderung der Metastasenbildung eines bösartigen Tumors in einem Patienten verwendet wird, der einer Operation zur Entfernung eines Tumors unterzogen wird und wobei der natriuretische Peptidrezeptor-GC-B-Agonist kontinuierlich, beginnend vor der Operation bis zu 1 oder mehreren Tagen nach der Operation, verwendet wird.

**5.** Natriuretischer Peptidrezeptor-GC-B-Agonist zur Verwendung nach Anspruch 1, wobei ein Peptid, bestehend aus einer der Aminosäuresequenzen der SEQ ID Nrn. 7 bis 10 kontinuierlich in einer Dosierung von 0,05 μg/kg/min oder weniger, beginnend vor der den Tumor entfernenden Operation, für einen gewissen Zeitraum nach der Operation verwendet wird.

**6.** Natriuretischer Peptidrezeptor-GC-B-Agonist zur Verwendung nach Anspruch 4, wobei ein Peptid, bestehend aus einer der Aminosäuresequenzen der SEQ ID Nrn. 7 bis 10, kontinuierlich in einer Dosierung von 0,025 μg/kg/min, beginnend während der Operation und fortgesetzt für 3 Tage nach der Operation verwendet wird.

**7.** Natriuretischer Peptidrezeptor-GC-B-Agonist zur Verwendung nach Anspruch 1, wobei zwei oder mehr Arten des natriuretischen Peptidrezeptor-GC-B-Agonisten als aktive Inhaltsstoffe in Kombination verwendet werden.

**8.** Natriuretischer Peptidrezeptor-GC-B-Agonist zur Verwendung nach Anspruch 1, wobei die Implantation und/oder die Invasion von Tumorzellen in vaskuläres endotheliales Gewebe in einem Patienten gehemmt wird, der den natriuretischen Peptidrezeptor-GC-B-Agonisten erhalten hat.

**9.** Natriuretischer Peptidrezeptor-GC-B-Agonist zur Verwendung nach Anspruch 1, der in einem Individuum verwendet wird, das gefährdet ist, einen epithelialen, bösartigen Tumor zu entwickeln, um dem Individuum mindestens einen pharmakologischen Effekt bereitzustellen, der ausgewählt ist aus der Unterdrückung der epithelialen zu mesenchymalen Transition (EMT) von Tumorzellen, der Inhibition des Erwerbs der Migrationsfähigkeit von Tumorzellen, der Unterdrückung der von Tumorzellen erworbenen Motilität, der Unterdrückung der Invasion durch Tumorzellen, der Induktion von tumorzellspezifischer Apoptose, und der Inhibition der Implantation von Tumorzellen in vaskuläres endotheliales Gewebe.

**10.** Natriuretischer Peptidrezeptor-GC-B-Agonist zur Verwendung nach Anspruch 1, wobei der Patient einer Operation zur Entfernung eines bösartigen Tumors unterzogen wird.

**11.** Natriuretischer Peptidrezeptor-GC-B-Agonist zur Verwendung nach Anspruch 10, wobei der Patient die Verabreichung des natriuretischen Peptidrezeptor-GC-B-Agonisten für mehrere Tage, beginnend mit dem Tag vor der Operation, erhält.

**12.** Natriuretischer Peptidrezeptor-GC-B-Agonist zur Verwendung nach Anspruch 10, wobei der Patient die Verabreichung des natriuretischen Peptidrezeptor-GC-B-Agonisten einen Tag vor der Operation erhält.

**13.** Natriuretischer Peptidrezeptor-GC-B-Agonist zur Verwendung nach Anspruch 10, wobei der natriuretische Peptidrezeptor-GC-B-Agonist dem Patienten intramuskulär oder subkutan verabreicht wird.

**14.** Natriuretischer Peptidrezeptor-GC-B-Agonist zur Verwendung nach einem der Ansprüche 1 bis 13, wobei der natriuretische Peptidrezeptor-GC-B-Agonist in Kombination mit Cisplatin verwendet wird.

**15.** Natriuretischer Peptidrezeptor-GC-B-Agonist zur Verwendung nach Anspruch 14, wobei der natriuretische Peptidrezeptor-GC-B-Agonist ein atriales natriuretisches Peptid ist, bestehend aus einer der Aminosäuresequenzen der SEQ ID Nrn. 7 bis 10.

**Revendications**

**1.** Agoniste du récepteur GC-B des peptides natriurétiques pour une utilisation dans la prévention ou la suppression de métastase d'une tumeur maligne, dans lequel l'agoniste du récepteur GC-B des peptides natriurétiques est un agoniste quelconque choisi parmi les agonistes (b1) à (b5) suivants ou un sel pharmacologiquement acceptable de ceux-ci et a une activité agoniste pour le récepteur GC-B des peptides natriurétiques :

(b1) un peptide natriurétique de type c constitué par la séquence d'acides aminés de SEQ ID No: 7, 8, 9 ou 10,
(b2) une substance comprenant un fragment actif, qui a la séquence d'acides aminés de SEQ ID No: 11, ou qui est constitué par la séquence d'acides aminés de la position 6 à la position 22 de SEQ ID No: 7,
(b3) un mutant qui est :

(i) un peptide constitué par la séquence d'acides aminés de SEQ ID No: 7, 9 ou 10 portant une substitution, délétion, insertion, et/ou addition d'un à plusieurs acides aminés choisis parmi les positions 1 à 5,
(ii) un peptide constitué par la séquence d'acides aminés de SEQ ID No: 8 portant une substitution, délétion, insertion, et/ou addition d'un à plusieurs acides aminés à une à plusieurs positions quelconques choisies parmi les positions 1 à 36,

(b4) un dérivé comprenant l'une quelconque des séquences d'acides aminés de SEQ ID No: 7 à 11, dans lequel de préférence le dérivé comprend en outre au moins une parmi la région Fc d'une immunoglobuline, une sérum albumine et l'extrémité C-terminale de la ghréline, et

(b5) une forme modifiée qui comprend l'une quelconque des séquences d'acides aminés de SEQ ID No: 7 à 10 du listage des séquences, et au moins un acide aminé autre que les acides aminés représentés dans SEQ ID No: 11 qui est chimiquement modifié, ou qui est préparée par modification chimique par addition d'un polymère pharmaceutiquement utilisable.

2. Agoniste du récepteur GC-B des peptides natriurétiques pour son utilisation selon la revendication 1, dans lequel l'agoniste du récepteur GC-B des peptides natriurétiques est utilisé en continu à raison de 0,5 $\mu$g/kg/min ou moins pendant trois à sept jours.

3. Agoniste du récepteur GC-B des peptides natriurétiques pour son utilisation selon la revendication 1, dans lequel l'agoniste du récepteur GC-B des peptides natriurétiques est utilisé en continu à raison de 0,1 $\mu$g/kg/min ou moins pendant un à plusieurs jours.

4. Agoniste du récepteur GC-B des peptides natriurétiques pour son utilisation selon la revendication 1, dans lequel l'agoniste du récepteur GC-B des peptides natriurétiques est utilisé pour supprimer ou prévenir la métastase d'une tumeur maligne chez un patient qui subit une ablation chirurgicale d'une tumeur et, dans lequel l'agoniste du récepteur GC-B des peptides natriurétiques est utilisé en continu depuis avant l'opération et jusqu'à 1 à plusieurs jours après.

5. Agoniste du récepteur GC-B des peptides natriurétiques pour son utilisation selon la revendication 1, dans lequel un peptide constitué par l'une quelconque des séquences d'acides aminés de SEQ ID No: 7 à 10 est utilisé en continu à une dose de 0,05 $\mu$g/kg/min ou moins depuis avant l'ablation chirurgicale de la tumeur et pendant un certain temps après.

6. Agoniste du récepteur GC-B des peptides natriurétiques pour son utilisation selon la revendication 4, dans lequel un peptide constitué par l'une quelconque des séquences d'acides aminés de SEQ ID No: 7 à 10 est utilisé en continu à une dose de 0,025 $\mu$g/kg/min pendant une période démarrant pendant l'opération et se poursuivant 3 jours après.

7. Agoniste du récepteur GC-B des peptides natriurétiques pour son utilisation selon la revendication 1, dans lequel deux types ou plus de l'agoniste du récepteur GC-B des peptides natriurétiques sont utilisés en combinaison, à titre de principes actifs.

8. Agoniste du récepteur GC-B des peptides natriurétiques pour son utilisation selon la revendication 1, dans lequel l'implantation et/ou l'invasion de cellules tumorales dans le tissu endothélial vasculaire est inhibée chez un patient qui a reçu l'agoniste du récepteur GC-B des peptides natriurétiques.

9. Agoniste du récepteur GC-B des peptides natriurétiques pour son utilisation selon la revendication 1, qui est utilisé chez un sujet à risque de développer une tumeur maligne épithéliale pour fournir au sujet au moins un effet pharmacologique choisi parmi la suppression de la transition épithéliale à mésenchymateuse (EMT) des cellules tumorales, l'inhibition de l'acquisition de la capacité de migration par les cellules tumorales, la suppression de l'acquisition de la motilité par les cellules tumorales, la suppression de l'invasion par les cellules tumorales, l'induction de l'apoptose spécifique des cellules tumorales, et l'inhibition de l'implantation des cellules tumorales dans le tissu endothélial vasculaire.

10. Agoniste du récepteur GC-B des peptides natriurétiques pour son utilisation selon la revendication 1, dans lequel le patient subit l'ablation chirurgicale d'une tumeur maligne.

11. Agoniste du récepteur GC-B des peptides natriurétiques pour son utilisation selon la revendication 10, dans lequel le patient reçoit une administration d'agoniste du récepteur GC-B des peptides natriurétiques pendant plusieurs jours à compter de la veille de l'opération.

12. Agoniste du récepteur GC-B des peptides natriurétiques pour son utilisation selon la revendication 10, dans lequel le patient reçoit une administration d'agoniste du récepteur GC-B des peptides natriurétiques la veille de l'opération.

13. Agoniste du récepteur GC-B des peptides natriurétiques pour son utilisation selon la revendication 10, dans lequel l'agoniste du récepteur GC-B des peptides natriurétiques est administré au patient par voie intramusculaire ou par voie sous-cutanée.

14. Agoniste du récepteur GC-B des peptides natriurétiques pour son utilisation selon l'une quelconque des revendications 1 à 13, dans lequel l'agoniste du récepteur GC-B des peptides natriurétiques est utilisé en combinaison avec le cisplatine.

15. Agoniste du récepteur GC-B des peptides natriurétiques pour son utilisation selon la revendication 14, dans lequel l'agoniste du récepteur GC-B des peptides natriurétiques est un peptide natriurétique atrial constitué par l'une quelconque des séquences d'acides aminés de SEQ ID No: 7 à 10.

[Fig. 1]

[Fig. 2]

[Fig. 3]

[Fig. 4]

[Fig. 5]

[Fig. 6]

A

B

[Fig. 7]

[Fig. 8]

[Fig. 9]

A

a                  b                  c

B

[Fig. 10]

[Fig. 11]

[Fig. 12]

[Fig. 13]

EP 3 189 835 B1

[Fig. 14]

[Fig. 15]

[Fig. 16]

[Fig. 17]

[Fig. 18]

[Fig. 19]

[Fig. 20]

[Fig. 21]

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 2009067639 A
- US 20100310561 A
- WO 2002046227 A
- WO 2009142307 A
- US 2010305031 A
- US 20090175821 A
- US 7662773 B
- WO 91009968 A
- WO 9203918 A
- WO 9425585 A
- EP 17153823 A
- JP 2011041263 A

## Non-patent literature cited in the description

- **THOMAS R. GEIGER et al.** *Biochim. Biophys. Acta,* 2009, 293-308
- **SILVER MA.** *Curr. Opin. Nephrol. Hypertens.,* 2006, vol. 15, 14-21
- **YOSHIBAYASHI M. et al.** *Eur. J. Endocrinol.,* 1996, vol. 135, 265-268
- **XUNG J. et al.** *J. appl. Physiol.,* 2011, vol. 110 (1), 213-224
- **VESELY BA et al.** *Eur J Clin Invest.,* 2005, vol. 35, 60-69
- **DAS et al.** *Proc. Natl. Acad. Sci.,* 2010, vol. 107 (42), 18202-18207
- **DIAN C. N. et al.** *Journal of Urology,* 2003, vol. 170 (3), 994-997
- **LUBBE W. J. et al.** *Cancer Res.,* 2009, vol. 69 (8), 3529-3536
- **KONG L. et al.** *Clin Exp. Metastasi,* 1996, vol. 14 (3), 335-343
- **THOMAS R. GEIGER et al.** *Biochim. Biophys. Acta.,* 2009, 293-308
- **KONG L. et al.** *Clin Exp. Metastasis,* 1996, vol. 14 (3), 335-343
- **CHINKERS M et al.** *Nature,* 1989, vol. 338, 78-83
- *Biochem. Biophys. Res. Commun.,* 1984, vol. 118, 131
- **A. CALDERONE.** *Minerva Endocrinol.,* 2004, vol. 29, 113-127
- **SUGA S. et al.** *Endocrinology,* 1992, vol. 130 (1), 229-239
- **FURUYA, M. et al.** *Biochem. Biophys. Res. Commun.,* 1992, vol. 183 (3), 964-969
- *Biochem. Biophys. Res. Commun.,* 1984, vol. 121, 585
- *Medicinal Research Review,* 1990, vol. 10, 115
- *Science,* 1995, vol. 270, 1657
- **DEBORAH et al.** *J. Biol. Chem.,* 2008, vol. 289 (50), 35003-35009
- **CUCULI, E et al.** *Expert Opinn. Investig. Drugs,* 2011, vol. 20 (4), 457-463
- **MATTHEW I. et al.** *Br. J. Clin. Pharmacol.,* 2004, vol. 57 (1), 27-36
- **M. P. GOVANNONI et al.** *Curr. Med. Chem.,* 2010, vol. 17, 2564-2587
- **POTTER L. R.** *Pharmacology & Therapeutics,* 2011, vol. 130 (1), 71-82
- *J. Clin. Hypertens.,* 2006, vol. 8 (12), 40-52
- **R. P. MASON et al.** *J. Clin Hypertens.,* 2006, vol. 8 (S12), 40-52
- **CORBIN J. D. et al.** *J. Biol. Chem.,* 1986, vol. 261 (3), 1208-1214
- *Science,* 1984, vol. 226, 1206
- *Biochem. Biophys. Res. Commun.,* 1989, vol. 165, 650
- Jikken Igaku (Experimental Medicine). 1994, vol. 12, 303
- **DEBORAH et al.** *J. Biol. Chem.,* 2008, vol. 289 (50), 35003-35009
- **TOKUDOME T. ; KISHIMOTO I. ; KANGAWA K. et al.** *Arterioscler Thromb Vase Biol.,* 2009, vol. 29, 1516-21
- **KISHIMOTO I ; TOKUDOME T ; NAKAO K ; KANGAWA K.** *FEBS J.,* 2011, vol. 278, 1830-41
- **ZHUN ML et al.** *Cardiovasc Res.,* 2009, vol. 84, 292-9